# EUROPEAN PATENT APPLICATION

(11) **EP 1 022 029 A1**
(43) Date of publication of application: **26.07.2000**
(21) Application number: 98941864.5
(22) Date of filing: 14.09.1998
(51) Int. Cl.: A61K 45/00, A61K 31/40, C07D 207/27

(54) **NOOTROPIC AGENT**

(30) Priority: 12.09.1997 JP 24913197
(71) Applicant: DAIICHI PHARMACEUTICAL CO., LTD., Chuo-ku, Tokyo 103-8234 (JP)
(72) Inventor: NISHIZAKI, Tomoyuki, Hyogo 651-1232 (JP); YOSHII, Mitsunobu, Tokyo 156-0054 (JP); WATABE, Shigeo, Daiichi Pharmaceutical Co., Ltd., Edogawa-ku Tokyo 134-8630 (JP)
(74) Representative: Hartz, Nikolai F., Dr.
(86) International application number: JP9804136
(87) International publication number: WO9913911

(57) **Abstract**

A drug which improves cerebral neurotransmission based on interaction of long-term activation of a protein kinase C pathway and long-term activation of presynaptic nicotinic acetylcholine (nACh) receptors, and on an accompanying increase in the amount of released glutamate, which activates cerebral neurotransmission over a prolonged period. The substances of the present invention serve as excellent nootropics. They are particularly useful for the therapy of dementia caused by hydrocephalus, or by subdural hemorrhage.

## Description

### TECHNICAL FIELD

The present invention relates to nootropics, and more particularly to pharmaceuticals which act on the neurotransmission system in the brain for a prolonged period and thus are useful for the treatment of dementia, etc.

### BACKGROUND ART

Lines of evidence indicate that the neuronal nicotinic acetylcholine (nACh) receptors, which are preferentially localized on the presynaptic sites and involved in regulation of the release of neurotransmitters (K.F. Funk *et al.*, Biomed. Biochem. Acta 11, 1301, 1984: G. Spignoli *et al.*, Pharmacol. Biochem. Behav. 27, 491, 1987: M. Marchi *et al.*, Eur. J. Pharmacol. 185, 247, 1990: S. Watabe *et al.*, J. Pharmacol. 238, 303, 1993), facilitate hippocampal neurotransmission linked to cognitive function (S. M. Satoh *et al.* Neurosci. Lett. 68, 216, 1986).

Until now, almost no clinically-effective therapeutic drugs for dementia have been found. Recently, several types of nootropics have been developed; however, their specific mechanisms are unknown.

An object of the present invention is to provide a drug which provides long-term improvement of cerebral neurotransmission, based on an agent that facilitates hippocampal neurotransmission for a prolonged period, for treatment of various types of dementia such as those attributable to hydrocephalus, or Alzheimer's disease.

### DISCLOSURE OF THE INVENTION

Numerous studies have shown that a certain type of nootropics (or cognition-enhancing agents) can improve various neurotransmissions in the brain; those in the doperminergic, cholinergic, glutaminergic, and GABAergic systems.

Also, some nootropics are known to facilitate long-term potentiation (LTP) which is caused by activated neurotransmission in response to tetanic stimulation (electrical stimulation) (M. Satoh *et al.*, Neurosci. Lett. 68, 216, 1986). LTP serves as a model system of memory and learning. However, in actual medical practice, application of electrical stimulation (tetanic stimulation) is neither realistic nor clinically useful for inhibiting dementia.

In view of the foregoing, the present inventors made great efforts to discover a substance which activates neurotransmission over a long period of time without utilization of electrical stimulation (tetanic stimulation). As a result, they found that the following compounds are useful as drugs for the treatment of dementia resulting from a variety of causes, i.e., as nootropics, leading to completion of the invention:
(1) a substance which activates a protein kinase C-activation pathway over a long period of time,
(2) a substance which provides an interaction of long-term activation of protein kinase C and long-term activation of a nicotinic acetylcholine receptor;
(3) a substance which activates a protein kinase C-activation pathway over a long period of time, so as to activate a presynaptic nicotinic acetylcholine receptor based on an interaction with the protein kinase C, to thereby increase release of glutamate for a long period for the improvement of hippocampal neurotransmission over a long period of time; and
(4) a substance which evokes an LTP-like action in the absence of electrical stimulation.

Accordingly, the present invention provides a nootropic which contains as the active ingredient a substance which activates a protein kinase C-activation pathway over a long period of time.

The present invention also provides a nootropic which contains as the active ingredient a substance which provides an interaction of long-tern activation of protein kinase C and long-term activation of a nicotinic acetylcholine receptor.

The present invention also provides a nootropic which contains as the active ingredient a substance which activates a protein kinase C-activation pathway over a long period of time, so as to activate a presynaptic nicotinic acetylcholine receptor based on an interaction with the protein kinase C, to thereby increase release of glutamate for a long period for the improvement of hippocampal neurotransmission over a long period of time.

The present invention also provides a nootropic which contains as the active ingredient a substance which evokes an LTP-like action in the absence of electrical stimulation.

The present invention also provides an agent for long-term facilitation of cerebral neurotransmission; an agent for long-term facilitation of protein kinase C-activation pathway; an agent for long-term facilitation of protein kinase C-activation pathway; an agent for evoking an LTP-like action in the absence of electrical stimulation; and a long-term enhancer for glutamate release; each contraining, as an active ingredient, 2-oxo-1-pyrrolidinylalkylcarboxylic amide having the following formula (1): 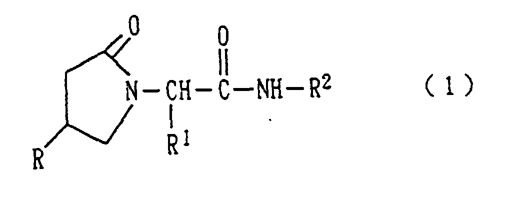 [wherein R represents a hydrogen atom or a hydroxyl group; R¹ represents a hydrogen atom or a methyl group; and R² represents a pyridyl group or a substituted phenyl group having 1 to 3 substituents which may be identical to or different from one another, wherein substituents on the phenyl group include
a halogen atom,
a trifluoromethyl group,
a nitro group,
an acetyl group,
a linear or branched C₁₋₄ alkyl group,
a linear or branched C₁₋₄ alkoxyl group,
a linear or branched C₁₋₇ alkylmercapto group,
a substituted alkylmercapto group represented by -S-(CH₂)ₙ-CH(R³)(R⁴) [wherein n represents 1 or 2; R³ represents a hydrogen atom or a methyl group; R⁴ represents a hydroxyl group or an amino group represented by -N(R⁸)(R⁹) (wherein R⁸ represents a hydrogen atom or a methyl group and R⁹ represents a methyl group, a benzyl group, or a substituted benzyl group, or R⁸ and R⁹ may be linked to each other and form a substituted pyrrolidine ring together with the nitrogen atom in the formula)],
a sulfonyl group represented by -SO₂R⁵ (wherein R⁵ represents an amino group or a C₁₋₃ alkyl group), and
a substituted aminoethoxycarbonyl group represented by -COO(CH₂)₂-N(R⁶)(R⁷) (wherein each of R⁶ and R⁷ represents a hydrogen atom, a methyl group, or an ethyl group)],
or pharmaceutically acceptable acid-addition salts thereof.

The present invention also provides use, in manufacture of nootropics, of a substance which activates a protein kinase C-activation pathway over a long period of time.

The present invention also provides use, in manufacture of nootropics, of a substance which provides an interaction of long-term activation of protein kinase C and long-term activation of a nicotinic acetylcholine receptor.

The present invention also provides use, in manufacture of nootropics, of a substance which activates a protein kinase C-activation pathway over a long period of time, so as to activate a presynaptic nicotinic acetylcholine receptor based on an interaction with the protein kinase C, to thereby increase release of glutamate for a long period for the improvement of hippocampal neurotransmission over a long period of time.

The present invention also provides use of the compound of formula (1) in manufacture of a long-term facilitator for cerebral neurotransmission, a long-term activator for protein kinase C-activation pathway, an inducer for an LTP-like action without need of electrical stimulation, or a long-term enhancer for glutamate release.

The present invention also provides a treatment method for enhancing cognition, characterized by administering to a subject a substance which activates a protein kinase C-activation pathway over a long period of time.

The present invention also provides a treatment method for enhancing cognition, characterized by administering to a subject a substance which provides an interaction of long-term activation of protein kinase C and long-term activation of a nicotinic acetylcholine receptor.

The present invention also provides a treatment method for enhancing cognition, characterized by administering to a subject a substance which activates a protein kinase C-activation pathway over a long period of time, so as to activate a presynaptic nicotinic acetylcholine receptor based on an interaction with the protein kinase C, to thereby increase release of glutamate for a long period for the improvement of hippocampal neurotransmission over a long period of time.

The present invention also provides a treatment method for enhancing cognition, characterized by administering to a subject a substance which evokes an LTP-like action without requiring electrical stimulation.

The present invention also provides a treatment method for long-term facilitation of cerebral neurotransmission, for activating protein kinase C-activation pathway; for evoking of an LTP-like action without requiring electrical stimulation; or for enhancing glutamate release for a prolonged period; each comprising the step of administering a compound of formula (1) to a subject.

### BRIEF DESCRIPTION OF THE DRAWINGS

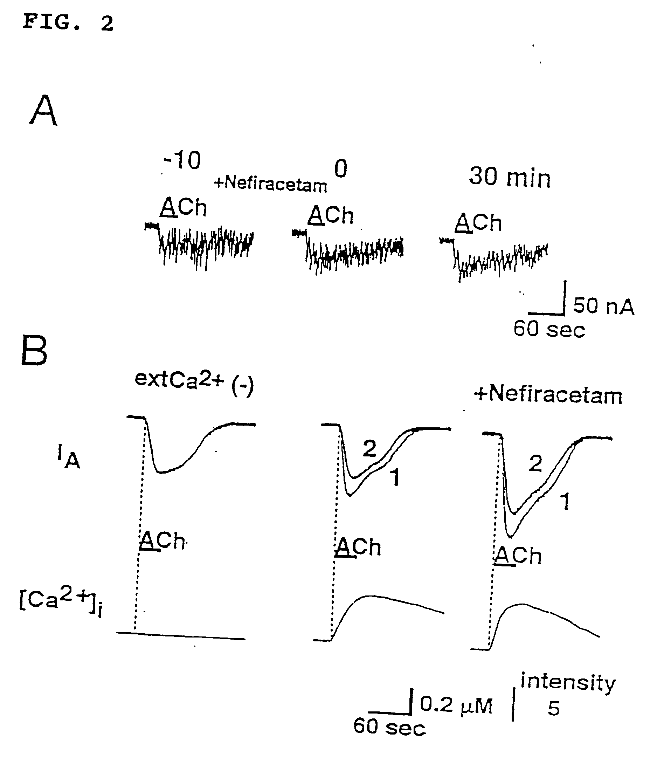

Fig. 1 shows the effect of nefiracetam on non-NMDA receptor currents. GluR 1, 2, 3 receptors which are AMPA receptors were expressed in *Xenopus* oocytes. Cell membrane currents evoked with kainate (100 µM) were recorded before and after administration of nefiracetam (1 µM) (n=5). The holding voltage was -30 mV.
Fig. 2 shows the effect of nefiracetam on Ca²⁺-dependent chloride currents and Ca²⁺ influx through the normal nicotinic ACh receptors.
   (A): In atropine-free frog Ringer's solution, acetylcholine (100 µM) was applied to an oocyte which had not expressed ACh receptors. The holding voltage was -30 mV.
   (B): An oocyte expressing normal ACh receptors was loaded with Calcium Green. In Ca²⁺-free (extCa²⁺(-)) extracellular solution (extCa²⁺(-)) and ordinary (i.e., Ca²⁺-containing) extracellular solution, intracellular Ca²⁺ ([Ca²⁺]ᵢ) and Ach-evoked currents (IA) were recorded. The Ca²⁺ rise was normalized by dividing "Δ increase of fluorescence intensity (DI)" by "base intensity (I)", and further by "current amplitude obtained with Ca²⁺-free solution (2) (µA)". Indicated by "(1)" are currents obtained with Ca²⁺-containing solution. In Fig. 2, each value represents the average from 7 oocytes and the errors in SD are indicated by bars.

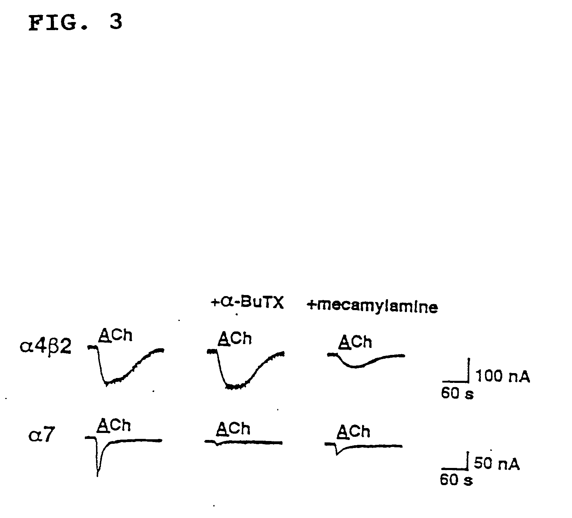

Fig. 3 shows potentiation of α4β2 and α7 receptor currents evoked by nefiracetam.

Two-electrode voltage-clamp technique was used to record expression α4β2 or α7 receptors in oocytes. ACh (100 µM) was applied to a single oocyte in the presence or absence of α-BuTX (50 nM) or MCA (3 µM). The holding voltage was -30 or -60 mV for α4β2-nAChR or α7-nAChR, respectively. Downward directions correspond to inward currents.

### BEST MODES FOR CARRYING OUT THE INVENTION

In search for a substance which provides a long-term potentiation of neurotransmission of the present invention, the following means are employed. .
(1) Hippocampal slices are prepared from the brain of an animal such as a rat by use of standard techniques. The slices are placed in a recording chamber containing an artificial cerebrospinal fluid saturated with proper amounts of oxygen and carbonic acid, to thereby perform continuous superfusion. The dendritic field excitatory postsynaptic potentials were recorded in the pyramidal cell layer of the CA1 region by electrical stimulation to the Schaeffer collateral/commisural fiber. That is, in this method, a substance that potentiates hippocampal neurotransmission is evaluated through measurement of the postsynaptic potentials.
(2) Another method uses *in vitro* transcription. In this method, mRNA receptors known as cerebral nACh receptors are obtained and injected into *Xenopus* oocytes, and cells expressing the receptors are used. For example, as nACh receptors, there are used rat α4β2 mRNA and α7 mRNA, and mRNAs of peptide NP152 having PKC-inhibitory functions and peptide NP153 that inhibits inactive PKC; and as AMPA receptors, there are used mRNAs of GluR 1, 2, 3 receptor mRNAs. After injection of the respective mRNAs into *Xenopus* oocytes, receptors or peptide is expressed. Subsequently, respective receptor currents are measured by use of the oocyte expressing the receptors.
(3) Yet another method identifies neurotransmission pathways through quantitation of released glutamate, a neurotransmitter.
(4) In the evaluation methods in the present invention, GF109203X may be used as a protein kinase C inhibitor, H89 as a protein kinase A inhibitor, α-bungarotoxin as an α7 receptor inhibitor, mecamylamine (MCAM) as an α4β2 receptor inhibitor, D-2-amino-5-phosphovaleric acid (APV) as a non-NMDA type glutamate receptor inhibitor, and 6,7-dinitroquinozalin-2,3-dione (DNQX) as a selective non-NMDA type glutamate inhibitor.

Through use of the above-mentioned methods, the substance according to the present invention was verified to serve as a long-term cerebral neurotransmission enhancing agent. The findings will next be described.

### [1] Potentiation of postsynaptic potential by use of the compounds of the present invention:

Whether a compound is a long-term neurotransmission improving agent or not can be confirmed through measurement of postsynaptic potentials. The compounds of the present invention have been confirmed to enhance postsynaptic potentials, based on the following facts. That is, when postsynaptic potentials were recorded from the pyramical cell layer of the CA1 region of a rat hippocampal slice treated with the compound of the present invention at a concentration of 0.01-10 µM, the compound potentiated postsynaptic potentials in a dose dependent fashion (Tables 1 and 2). On the other hand, the compounds of the present invention provided short-term inhibition at low concentrations (0.1 µM or less), and LTP-like long-term potentiation at high concentrations (1 µM or more), showing a dose-dependent biphasic effect (Table 18).

In experiments using *Xenopus* oocytes expressing α7 and α4β2 receptors―which are known as cerebral nicotinic acethylcholine (hereinafter called simply "nACh") receptors―in treatment with the substance of the present invention, the potentiation of ACh-evoked currents was confirmed at a concentration of 0.001 µM or more (the maximum was 0.1 µM) in the case of the α4β2 receptor, and at a concentration of 0.01 µM or more (the maximum was 1 µM) in the case of the α7 receptor (Tables 7 and 8).

### [2] Confirmation of activation of protein kinase C (hereinafter called simply "PKC"):

PKC is a serine-threonine oxidation enzyme activated in the presence of calcium and phospholipid (phosphatidyl serine; PS), which was found by Nishizuka *et al.* in 1977. When the metabolic turnover of cell membrane inositol phospholipid is accelerated by various outside signals, two types of secondary messengers, i.e., DG (diacyl glycerol) and inositol triphosphate, are produced. The inositol triphosphate releases calcium from the calcium storage within the cell, to thereby increase the intracellular calcium concentration. In contrast, DG activates PKC in the presence of calcium and phospholipid. That is, cooperative action of PKC and calcium for phosphorylation regulates a variety of cell functions (Naoyosi Saitoh, Metabolism, 28: 189-222).

As mentioned above, the substance of the present invention exhibited potentiated postsynaptic potentials. The potentiation of postsynaptic potentials can be proven to be attributable to PKC activation through an experiment using hippocampal slices treated with GF109203X, a PKC inhibitor, and measurement of nACh receptor currents from a *Xenopus* oocyte membrane expressing nACh receptors.

The fact that the potentiation of postsynaptic potential is significantly inhibited by the substance of the present invention but not by protein kinase A (hereinafter called simply as "PKA"), a selective inhibitor, shows that the substance of the present invention affects the potentiation of postsynaptic potential.

In experiments using *Xenopus* oocyte membrane expressing α7 and α4β2 receptors, ACh-evoked currents were inhibited by the PKC inhibitor. In addition, the substance of the present invention suppressed the potentiation of ACh-evoked currents when the receptors were co-expressed with PKC-inhibitory peptide (NP152; αPKCl: Peunova, N. *et al.,* Nature 364, 450-453, 1993), and also suppressed potentiation of ACh-evoked currents when the receptors were co-expressed with inactive PKC-inhibitory peptide (NP153;ipKPC) (Table 10).

These facts indicate that the long-term potentiation of ACh-evoked currents caused by the treatment with the substance of the present invention is regulated by the variation of PKC activation.

### [3] Confirmation of activation of nACh receptors:

Studies conducted according to the above-mentioned methods show that the substance of the present invention activates nACh receptors as described below.

When neurons of the hippocampal CA1 region were treated with the substance of the present invention (1 µM), potentiation of postsynaptic potentials induced from the hippocampal CA1 region was suppressed by α-bungarotoxin (selective α7 receptor inhibitor) and mecamylamine (α4β2 receptor inhibitor). This indicates that the substance of the present invention activated nACh receptors (Tables 3 and 4).

The substance of the present invention potentiated ACh-evoked currents in the cells having α7 and α4β2 receptors, which are currently known cerebral nACh receptors (Table 8).

These results indicate that the substance of the present invention provides a long-lasting improvement of postsynaptic potentials through the neuronal nACh receptor.

Furthermore, the substance of the present invention (1 µM) exhibited no current potentiation in the mutant ACh receptors lacking potent PKC phosphorylation sites on the α and δ subunits (mαΔPKC/Ser333m δΔPKC/Ser377) (V. M. Gehle *et al.*, Mol. Brain Res. 5, 183, 1991) (Table 17). These results indicate that the substance of the present invention potentiated ACh-gated channel currents by activation of Ca²⁺-dependent PKC, and PKC phosphorylation of the receptors.

As mentioned above, it is indicated that the long-term facilitation of neurotransmission (potentiation of postsynaptic potentials) by the substance of the present invention is based on an interaction of long-term activation of PKC and that of nACh receptors.

### [4] Increase of released glutamate:

In an experiment using hippocampal slices, the substance of the present invention (1 µM) increased a glutamate release significantly under conditions where excitatory neurotransmitter glutamate is released under depolarization by means of electrical stimulation; however, the increase was inhibited by the presence of α-bungarotoxin or mecamylamine (Table 11). This indicates that the substance of the present invention activates nACh receptors, and thus increases a glutamate release, to thereby provide long-term potentiation of postsynaptic potentials.

### [5] Effects on AMPA/kainate receptor currents:

The source of postsynaptic potentials is α-amino-3-hydroxy-5-methyl-4-isoxazolopropionate (hereinafter called simply "AMPA")/kainate receptor currents.

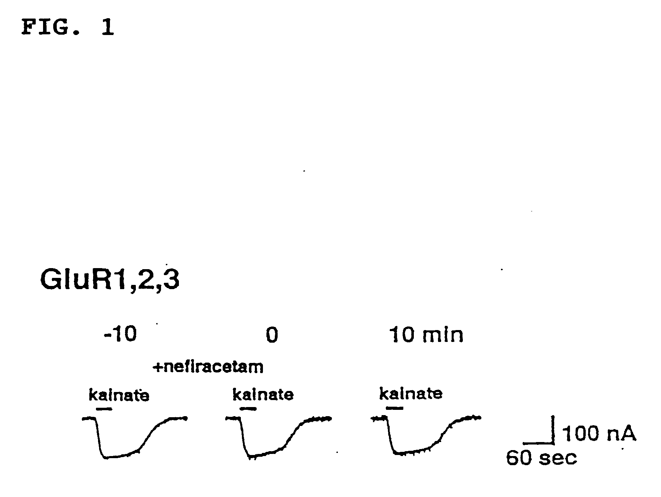

The facilitation of hippocampal neurotransmission by the substance of the present invention is inhibited by a selective inhibitor for the AMPA/kainate receptor (non-NMDA-type receptor) (DNQX) but is not inhibited by a selective inhibitor for the NMDA-type receptor (APV; 6,7-diphosphovaleric acid). This indicates that the "potentiation of postsynaptic potentials" by the substance of the present invention is attained through kainate receptor currents. However, in a study using a *Xenopus* oocyte expressing AMPA/kainate receptors (GluR 1, 2, 3), which are non-NMDA-type receptors (FIG. 1), the substance of the present invention did not potentiate AMPA/kainate receptor currents, indicating that the "potentiation of postsynaptic potentials" was not caused by modulation of the AMPA/kainate receptors expressed at the postsynaptic membrane.

### [6] Effects on ACh-gated channel currents:

ACh-evoked currents in the *Xenopus* oocyte membrane expression system are composed of ACh-gated channel (channel having an open-close mechanism modulated by ACh) currents and secondarily activated calcium-dependent chloride currents (R. Miledi *et al.*, J. Physiol. 357, 173, 1984). However, according to the studies conducted by the present inventors, the substance of the present invention had no effect on either calcium-dependent chloride currents induced by stimulation of the endogenous muscarinic ACh receptors (FIG. 2) or the calcium permeability of the nACh receptor channel (FIG. 2; Table 15). This indicates that the substance of the present invention modulated ACh-gated channel currents but not calcium(Ca²⁺)-sensitive chloride (Cl⁻) currents.

### [7] Biphasic effects:

In measurement of postsynaptic potentials from hippocampal slices, the substance of the present invention at a low concentration (≤0.1 µM) transiently exhibited a slight inhibition of postsynaptic potentials (Table 13).

The substance of the present invention, when administered perorally, exhibited significant, long-term potentiation action on *in vivo* cerebral excitatory postsynaptic potentials (PS) (Table 24).

The present invention will next be described in more detail.

The present invention is directed to a nootropics containing a substance activating a PKC activation pathway for a prolonged period as the active ingredient.

The present inventors have found that a drug that activates PKC for a prolonged period can serve as a nootropic. That is, the present inventors have found that, through long-term activation of PKC, particularly PKC which is related to the presynaptic nicotinic acetylcholine (nACh) receptor, nACh receptors are activated for a prolonged period by phosphorylation, to thereby promote release of glutamate by mediation of an intracellular transmission system, and the thus-released glutamate activates AMPA/kainate-type glutamate receptor and NMDA-type glutamate receptor, and these receptors cause Ca⁺⁺ and Na⁺⁺ influx into cells, leading to facilitation of neurotransimission.

This is also suggested by the results that the potentiation of postsynaptic potential exhibited in the presence of the substance of the present invention was not observed in the presence of a selective PKC inhibitor (Table 5).

The present invention is also directed to a nootropic which is characterized by its long-term improvement of hippocampal neuronal transmission caused by long-lasting activating of presynaptic nACh receptors through long-term activation of a PKC activation pathway.

The fact that nACh receptors are involved in potentiation of neurotransmission by the substance of the present invention is demonstrated by the test results in which postsynaptic potentials are inhibited by α-bungarotoxin (α-BuTX) (Table 3). Also, tests performed by the present inventors have proven that interaction of long-term activation of a PKC activation pathway and long-term activation of nACh receptors provides the long-term facilitation of hippocampal neurotransmission. These results indicate that the substance of the present invention is a nootropic which improves hippocampal neurotransmission through interaction of long-term activation of a PKC activation pathway and long-term activation of nACh receptors.

The present invention is also directed to a nootropic which contains as the active component a substance which activates a PKC-activation pathway over a long period of time, so as to activate a presynaptic nicotinic acetylcholine (nACh) receptor for a prolonged period, to thereby increase release of glutamate for a long period for the improvement of hippocampal neurotransmission over a long period of time.

The fact that nACh receptors participate in potentiation of neurotransmission caused by the substance of the present invention is suggested by, for example, the test results in which postsynaptic potentials are strongly inhibited by α-bungarotoxin (α-BuTX) (Table 3). Also, tests performed by the present inventors have proven that interaction of long-term activation of PKC and long-term activation of nACh receptors provides the long-term promotion of gulutamate release (Table 11). These results indicate that the substance of the present invention is a nootropic which ultimately improves hippocampal neurotransmission for a prolonged period through the above-described pathway and mechanism.

The present invention is also directed to a nootropic containing as the active ingredient a substance which evokes an LTP-like action while requiring no electrical stimulation.

As used herein, LTP stands for "long term potentiation" (hereinafter abbreviated as LTP), which refers to a state in which cerebral neurotransmission is activated over a long period of time, specifically a phenomenon in which postsynaptic potentials larger than those as observed before tetanic stimulation are observed for a long period (0.5-10 hours) when presynaptic fibers are subjected to high-frequency electrical stimulation (tetanic stimulation) and thereafter to a single stimulus, i.e., a long-term facilitation of synaptic neurotransmission via tetanic stimulation (*New-Cerebral Receptors* by Norio Ogawa, World Press, No. 296-299).

The expression "LTP-like action" refers to a phenomenon related to the nootropic of the present invention, in which postsynaptic potential similar to LTP is observed over a prolonged period of time. The significance of the LTP-like action is that this action can be evoked by the nootropic agent of the present invention alone, and thus application of tetanic stimulation is not needed. Moreover, although postsynaptic potantial evoked thereafter is similarly activated, it is unknown as to whether the biochemical process before evoking the postsynaptic potantial is also the same. In other words, although whether the LTP action and the LTP-like action are identical to each other or not is unclear, the substance of the present invention is unique in that it provides prolonged potentiation of postsynaptic potential, which potentiation is analogous to the LTP action, without need of tetanic stimulation (i.e., by the treatment with the substance of the present invention alone).

LTP action is considered to represents a long-term facilitation of neurotransmission, which is a model of plasticity formation of the central nervous system in terms of memory and learning, and based on this, it is accepted that the LTP action is involved in a variety of psychoneuronal diseases such as epilepsy, ischemic cerebral disorders, Alzheimer's disease, etc. From this, LTP-like action is also considered to exert the same effect. Accordingly, the substance of the present invention which evokes an LTP-like action is a potential therapeutic and preventive drug for these neuro-psychiatric diseases.

The nootropic of the present invention is also useful as a therapeutic drug for dementia caused by chronic subdural hemorrhage and as a therapeutic drug for dementia caused by hydrocephalus.

Since in dementia caused by chronic subdural hemorrhage and in that caused by normal pressure hydrocephalus it is considered that cerebral neurotransmission is damaged or its functions are lowered, the nootropic of the present invention, which is expected to improve neurotransmission over a prolonged period of time, is advantageously used as a preventive and therapeutic agent of these diseases.

The active ingredient of the above-described nootropic of the present invention is 2-oxo-1-pyrrolidinylalkylcarboxylic amide having the following formula (1): 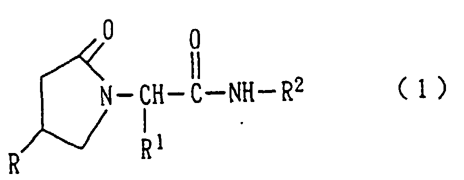 [wherein R represents a hydrogen atom or a hydroxyl group; R¹ represents a hydrogen atom or a methyl group; and R² represents a pyridyl group or a substituted phenyl group having 1 to 3 substituents which may be identical to or different from one another, wherein substituents on the phenyl group include
a halogen atom,
a trifluoromethyl group,
a nitro group,
an acetyl group,
a linear or branched C₁₋₄ alkyl group,
a linear or branched C₁₋₄ alkoxyl group,
a linear or branched C₁₋₇ alkylmercapto group,
a substituted alkylmercapto group represented by -S-(CH₂)ₙ-CH(R³)(R⁴) [wherein n represents 1 or 2; R³ represents a hydrogen atom or a methyl group; R⁴ represents a hydroxyl group or an amino group represented by -N(R⁸)(R⁹) (wherein R⁸ represents a hydrogen atom or a methyl group and R⁹ represents a methyl group, a benzyl group, or a substituted benzyl group, or R⁸ and R⁹ may be linked to each other and form a substituted pyrrolidine ring together with N in the formula)],
a sulfonyl group represented by -SO₂R⁵ (wherein R⁵ represents an amino group or a C₁₋₃ alkyl group), and
a substituted aminoethoxycarbonyl group represented by -COO(CH₂)₂-N(R⁶)(R⁷) (wherein each of R⁶ and R⁷ represents a hydrogen atom, a methyl group, or an ethyl group)]
or a pharmaceutically acceptable salt thereof.

Examples of the substituents of phenyl group include a halogen atom such as a chlorine atom or a fluorine atom; an alkyl group such as a methyl group, an ethyl group, an n-propyl group, a sec-butyl group, and an n-butyl group; an alkoxy group such as a methoxy group or an isopropoxy group; an alkylmercapto group such as a methylmercapto group, an n-propylmercapto group, an isopropylmercapto group, a sec-butylmercapto group, or an n-heptylmercapto group; a substituted mercapto group such as a 2-hydroxypropylmercapto group, a 2-(N,N-dimethylamino)propylmercapto group, or a 2-(N-methyl-N-benzylamino)ethylmercapto group; an N-methyl-N-benzylaminoalkylmercapto group having a formula of -S-(CH₂)ₙ-CH(R³)-N(R⁸)(R⁹) wherein a substituted benzyl group represented by R⁹ is a benzyl group substituted with a methoxy group, such as, for example, a 2-N-methyl-N-(3,4-dimethoxybenzyl)aminoethylmercapto group;, a 1-pyrrolidinylalkylmercapto group having a formula of -S-(CH₂)ₙ-CH(R³)-N(R⁸)(R⁹) wherein a pyrrolidinyl ring formed of R⁸ and R⁹ is substituted with a 2-oxo group, such as, for example, a 2-(2-oxo-1-pyrrolidinyl)ethylmercapto group; and a 2-(N,N-diethylamino)ethoxycarbonyl group. Either a pyridyl group represented by R² or a 4-pyridyl group may be used.

Examples of another group of compounds suitable for the therapeutic drugs of the present invention include:
1. 2-oxo-1-pyrrolidinylacetic acid 2,6-dimethylanilide;
2. 4-hydroxy-2-oxo-1-pyrrolidinylacetic acid 2,6-diethylanilide;
3. 4-hydroxy-2-oxo-1-pyrrolidinylacetic acid 2,6-dimethylanilide;
4. 2-[2-oxopyrrolidinyl]propionic acid N-3-pyridinylamide;
5. 2-oxo-1-pyyrolidinylacetic acid 4-isopropylmercaptoanilide;
6. 2-[2-oxo-1-pyrrolidinyl]-1-propionic acid 4-(2-butylmercapto)anilide;
7. 2-[2-oxo-1-pyrrolidinyl]propionic acid 4-isopropylanilide;
8. 2-[2-oxo-1-pyrrolidinyl]propionic acid 2,4-dimethylanilide;
9. 2-[2-oxo-1-pyrrolidinyl]propionic acid 2,4,6-methoxy-5-methylanilide;
10. 2-[2-oxo-1-pyrrolidinyl]propionic acid 2-methoxy-5-methylanilide;
11. 2-[2-oxo-1-pyrrolidinyl]propionic acid 2,6-dichloroanilide;
12. 2-pyrrolidoneacetamide;
13. 1-anisoyl-2-pyrrolidinone; and
14. 4-hydroxy-2-oxo-1-pyrrolidineacetamide.
Moreover, the compounds disclosed in Table 3 of Japanese Patent Publication (*kokoku*) No. 3-46466 (p 1019) also serve as useful compounds as active ingredients of the therapeutic drugs of the present invention.

Among the compounds which belong to the above-listed second group of compounds, there are particularly preferred 2-oxo-1-pyrrolidinylacetic acid 2,6-dimethylanilide 〈N-(2,6-dimethylphenyl)-2-(2-oxo-1-pyrrolidinyl)acetamide; non-proprietary name: nefiracetam〉; 2-pyrrolidone-acetamide; 1-anisoyl-2-pyrrolidinone; and 4-hydroxy-2-oxo-1-pyrrolidine-acetamide.

The compounds included in the above-listed two groups of compounds are all known, and these may easily be prepared by methods described in Japanese Patent Application Laid-Open (*kokai*) Nos. 56-2960, 61-280470, and 4-160496 and the like. The pyrrolidinylacetamide derivatives disclosed in Japanese Patent Publication (*kokoku*) No. 3-46466 are known to have a variety of biological actions, including cerebral function enhancing action (Japanese Patent Publication (*kokoku*) No. 62-5404), improving action for Alzheimer-type dementia (Japanese Patent Application Laid-Open (*kokai*) No. 5-163144), improving action for cerebrovascular dementia (Japanese Patent Application Laid-Open (*kokai*) No. 5-163145), anti-convulsion action in EL mice (Nakamoto *et al.*, The Seventeenth Conference of Japanese Society of Neurology, 1993, December 7 - December 9, in Nagoya, program abstract p. 84, presentation No. P1A20), and stabilizing action for mitochondrial membranes (Japanese Patent Application No. 8-260649). However, LTP-like action of those compounds (action exerted in the absence of tetanic stimulation) has heretofore been unknown.

Due to actions as described in [1] through [7] above, the compound of the present invention is useful as a cerebral neurotransmission enhancing agent; a long-term enhancer for glutamate release; a therapeutic drug for treatment of various types of dementia such as those attributable to hydrocephalus, chronic subdural hemorrhage, etc.; an LTP-like action-inducer requiring no electrical stimulation; and a long-term enhancer for protein kinase C.

Of the compounds represented by formula (1), nefiracetam is particularly useful for the above purposes. That is, nefiracetam is particularly useful as a long-term activator for a protein kinase C activation pathway. In short, presynaptic nAc receptors, when phosphorylated by PKC for a prolonged period, are activated for a prolonged period, to thereby induces―via intracellular transmission system―long-term increase of glutamate release, to thereby facilitate the neurotransmission of the hippocampal CA1 region through AMPA/kainate-type glutamate receptors and NMDA-type glutamate receptors, ultimately serving as a therapeutic drug for dementia.

Nefiracetam is useful as an inducer for an LTP-like action without need of electrical stimulation.

Nefiracetam is also useful as a long-term glutamate release enhancer based on interaction between a long-term activation of a PKC activation pathway and that of presynaptic nicotinic acetylcholine receptors.

Briefly, as shown in Examples below, nefiracetam is a compound which activates PKC for a prolonged period (Tables 5 and 7), activates presynaptic nACh receptors for a prolonged period (Tables 5, 8, 9, and 17), and augments release of a messenger glutamate for a prolonged period (Table 11).

Nefiracetam is useful as a long-term facilitator for cerebral neurotransmission based on interaction of long-lasting activation of protein kinase C activation pathway and long-lasting activation of presynaptic nicotinic acetylcholine (nACh) receptors.

As shown in Examples below, nefiracetam activates PKC for a prolonged period (Tables 5 and 7), activates presynaptic nACh receptors for a prolonged period (Tables 5, 8, 9, and 17), and augments release of a messenger glutamate for a prolonged period (Table 11), to thereby facilitate cerebral neurotransmission over a prolonged period. Thus, nefiracetam is a drug having an action of facilitating cerebral neurotransmission over a prolonged period.

The fact that the above-described effects were observed in animals to which nefiracetam was administered (Table 24) suggests utility of the compound of the present invention in humans.

Also, since the long-lasting facilitation of neurotransmission resembles LTP action, it is cosidered as an LTP-like action.

Neferacetam is useful as a therapeutic drug for dementia caused by brain chronic subdural hemorrhage based on an LTP-like action evoked without need of electrical stimulation, caused by long-term activation for PKC activation pathway.

Neferacetam is useful as a therapeutic drug for dementia caused by hydrocephalus based on an LTP-like action evoked without need of electrical stimulation, caused by long-term activation for PKC activation pathway.

Among various types of hydrocephalus which cause dementia, nefiracetam is particularly useful as a therapeutic drug for dementia caused by normal pressure hydrocephalus.

Neferacetam is useful as a nootropic, characterized by its long-term facilitation of hippocampal neurotransmission induced by long-term augmentation of glutamate release, based on interaction of long-term activation of a PKC activation pathway and presynaptic nACh receptors.

In this case, the word "nootropic" refers to a therapeutic drug for a variety of dementia, for Alzheimer's disease, and a drug useful for administration to subjects in need of facilitation or improvement of cerebral metabolic functions.

Nefiracetam is useful as a long-term enhancer for release of glutamate.

Nefiracetam has been confirmed to augment release of glutamate, which is a messenger in neurotransmission pathways, for long periods. Therefore, nefiracetam is said to be an agent which enhances gultamate release for a prolonged period.

Due to the enhanced glutamate release, hippocampal neurotransmission can be improved for a prolonged period, to thereby make nefiracetam a useful therapeutic and preventive agent for subjetcs who have symptoms with reduced cerebral neurotransmission, who partially lack cerebral neurotransmission, and who require long-lating improvement of hippocampal neurotransmission.

Both free-form compounds and physiologically acceptable salts thereof may be used as the therapeutic drugs of the present invention. Arbitrary hydrates or solvated forms thereof may also be used. Examples of the physiologically acceptable salts include acid-addition salts, e.g., inorganic acid salts such as hydrochlorides, hydrobromides, hydroiodides, sulfates, nitrates, and phosphates; and organic acid salts such as M acetates, maleates, fumarates, citrates, oxalates, succinates, tartarates, malates, mandelates, methanesulfonates, p-toluenesulfonates, and 10-camphor-sulfonates.

No particular limitation is imposed on the configuration of one or more asymmetric carbons in the compounds used as the active ingredient of the pharmaceuticals of the present invention, and arbitrary optically active species, arbitrary mixtures of optical isomers, or racemic modification may be used. Arbitrary mixtures of diastereoisomers having two or more asymmetric carbons may also be used.

No particular limitation is imposed on the manner of administration of the pharmaceuticals of the present invention, which may be administered perorally or parenterally. A compound serving as the active ingredient, e.g., a compound represented by the above formula (1), may be used as such, and preferably there is provided a pharmaceutical composition containing the compound that serves as an active ingredient and pharmacologically and pharmaceutically acceptable additives for pharmaceutical preparation. Examples of the pharmacologically and pharmaceutically acceptable additives which may be used include a vehicle, a disintegrant or its aid, a binder, a lubricant, a coating agent, a colorant, a diluting agent, a base, a solubilizer or its adjuvant, an isotonic agent, a pH-regulator, a stabilizer, a propellant, and a tackifying agent. Examples of the pharmaceutical preparations suitable for oral administration include tablets, capsules, powders, fine granules, granules, liquids, and syrups. Examples of the pharmaceutical preparations suitable for parenteral administration include injections, drops, suppositories, inhalants, and patches. To the pharmaceuticals of the present invention, one or more active ingredients may be incorporated.

No particular limitation is imposed on the dose of administration of the pharmaceuticals of the present invention, and they may be administered at a dose selected according to a variety of conditions such as purposes of treatment or prevention, type of disease, age or symptoms of patients, and a route of administration. Typically they are orally administered to an adult in a daily dose of approximately 19 mg - 1,000 mg, preferably approximate 60 - 900 mg, per day. 2-Oxo-1-pyrrolidinylacetic acid 2,6-dimethylanilide (non-proprietary name; nefiracetam), which is particularly preferably used in the present invention, has an acute toxicity of 2,005 mg/kg (male mouse, perorally), and thus is found to be quite safe (Japanese Patent Application Laid-Open (*kokai*) No. 163144).

### EXAMPLES

The present invention will next be described in detail by way of examples, which should not be construed as limiting the invention thereto.

### Example 1

### 1. Measurement of postsynaptic potential (Table 1)

Hippocampal slices (400 µm) were prepared from the rat brain by use of standard techniques. The slices were transferred to a recording chamber continuously superfused with 34°C artificial cerebrospinal fluid (in mM: 125 NaCl, 5 KCl, 1.24 KH₂PO₄, 1.3 MgCl₂, 2 CaCl₂, 26 NaHCO₃, 10 glucose) saturated with 95% O₂ and 5% CO₂ gases. The dendritic field excitatory postsynaptic potentials were recorded in the pyramidal cell layer of the CA1 region by the application of electrical stimulation (0.2 Hz) of the Schaffer collateral/commissural pathway (Table 1).

Hippocampal slices (400 µm) were prepared from the rat brain by use of standard techniques. The slices were transferred to a recording chamber continuously superfused with artificial cerebrospinal fluid saturated with a predetermined quantity of O₂ and CO₂ gases. Treatment with nefiracetam (0.01-10 µM) dose-dependently facilitated the postsynaptic potentials evoked from the hippocampal CA1 region (Table 2). The maximum facilitation was observed 60 minutes after the 1 µM treatment, and the magnitude was approximately 150% (Table 1, Table 2). This effect was inhibited by α-bungarotoxin (α-BuTX), a selective neuronal α 7nACh receptor antagonist (Table 3). This effect was also inhibited by mecamylamine, a non-selective neuronal nACh receptor antagonist; however, the degree of inhibition was less than that provided by α-bungarotoxin (Table 4). These results indicate that long-lasting facilitation of the postsynaptic potential is induced by nefiracetam via a neuronal nACh receptor.

Nefiracetam never potentiated the postsynaptic potential in the presence of GF109203X, a selective PKC inhibitor (Table 5). In contrast, H89, a selective inhibitor for cAMP-dependent PKA, showed no such effect (Table 5). The results indicate that long-lasting potentiation of the postsynaptic potential induced by nefiracetam is regulated by activation of PKC.

**Table 1**

| Effect of nefiracetam on postsynaptic potential of a slice of the rat hippocampus (change over time) | | | | | |
|---|---|---|---|---|---|
| Time (min) | Postsynaptic potential (% of the original amplitude) | | | | |
| | Mean ± SD | Treatment | (min) | Mean ± SD | Treatment |
| -30 | 100± 3 | | 6 | 131± 9 | |
| -10 | 100± 0 | ←nef. | 8 | 135± 9 | |
| -9 | 108± 1 | ←nef. | 10 | 143± 2 | |
| -8 | 109± 7 | ←nef. | 15 | 144± 1 | |
| -7 | 111± 2 | ←nef. | 20 | 142± 12 | |
| -6 | 122± 8 | ←nef. | 25 | 144± 8 | |
| -5 | 133± 6 | ←nef. | 30 | 140± 10 | |
| -4 | 127± 4 | ←nef. | 35 | 141± 10 | |
| -3 | 132± 7 | ←nef. | 40 | 155± 5 | |
| -2 | 132± 7 | ←nef. | 45 | 153± 9 | |
| -1 | 131± 9 | ←nef. | 50 | 155± 10 | |
| 0 | 131± 8 | | 55 | 156± 7 | |
| 2 | 136± 9 | | 60 | 156± 6 | |
| 4 | 129± 6 | | | | |
| The description "nef." indicates treatment with 1 µM nefiracetam (n=7). | | | | | |

**Table 2**

| Effect of nefiracetam on postsynaptic potential of a slice of the rat hippocampus (dose dependency) | |
|---|---|
| nefiracetam (µM) | Postsynaptic potential (% of original amplitude) |
| | Mean ± SD |
| 0.001 | 95 ± 6 |
| 0.01 | 94 ± 5 |
| 0.1 | 111 ± 9 |
| 1 | 156 ± 6 |
| 10 | 144 ± 11 |
| (n = 5 to 7) | |

**Table 3**

| Effect of bungarotoxin on nefiracetam-potentiated postsynaptic potential of a slice of the rat hippocampus | | | | | |
|---|---|---|---|---|---|
| Time (min) | Postsynaptic potential (% of original amplitude) | | | | |
| | Mean ± SD | Treatment | (min) | Mean ± SD | Treatment |
| -30 | 100 ± 5 | | 3 | 88 ± 6 | ←BuTX |
| -10 | 100 ± 0 | ←nef. | 4 | 79 ± 10 | ←BuTX |
| -9 | 114 ± 4 | ←nef. | 5 | 77 ± 6 | |
| -8 | 121 ± 1 | ←nef. | 6 | 86 ± 7 | |
| -7 | 121 ± 5 | ←nef. | 7 | 84 ± 8 | |
| -6 | 121 ± 6 | ←nef. | 8 | 86 ± 3 | |
| -5 | 129 ± 9 | ←nef. | 9 | 89 ± 4 | |
| -4 | 129 ± 5 | ←nef. | 10 | 96 ± 9 | |
| -3 | 132 ± 3 | ←nef. | 11 | 144 ± 5 | |
| -2 | 132 ± 7 | ←nef. | 12 | 132 ± 3 | |
| -1 | 132 ± 4 | ←nef. | 13 | 139 ± 9 | |
| 0 | 135 ± 6 | ←BuTX | 14 | 140 ± 6 | |
| 2 | 90 ± 5 | ←BuTX | 15 | 147 ± 7 | |
| nef. ; 1 µM nefiracetam BuTX ; 100 nM α-bungarotoxin n ; 5 | | | | | |

**Table 4**

| Effect of mecamylamine on nefiracetam-potentiated postsynaptic potential of a slice of the rat hippocampus | | | | | |
|---|---|---|---|---|---|
| Time (min) | Postsynaptic potential (% of original amplitude) | | | | |
| | Mean ± SD | | (min) | Mean ± SD | |
| -30 | 100 ± 7 | | 3 | 124 ± 8 | ←meca. |
| -10 | 100 ± 0 | ←nef. | 4 | 129 ± 4 | ←meca. |
| -9 | 108 ± 7 | ←nef. | 5 | 129 ± 9 | |
| -8 | 113 ± 5 | ←nef. | 6 | 137 ± 9 | |
| -7 | 123 ± 10 | ←nef. | 7 | 137 ± 5 | |
| -6 | 136 ± 10 | ←nef. | 8 | 139 ± 5 | |
| -5 | 137 ± 9 | ←nef. | 9 | 143 ± 5 | |
| -4 | 137 ± 9 | ←nef. | 10 | 145 ± 6 | |
| -3 | 140 ± 10 | ←nef. | 11 | 150 ± 7 | |
| -2 | 139 ± 8 | ←nef. | 12 | 150 ± 8 | |
| -1 | 141 ± 7 | ←nef. | 13 | 153 ± 9 | |
| 0 | 144 ± 8 | ←meca. | 14 | 154 ± 5 | |
| 1 | 128 ± 10 | ←meca. | 15 | 155 ± 4 | |
| 2 | 123 ± 7 | ←meca. | | | |
| nef. ; 1 µM nefiracetam meca. ; 3 µM α-mecamylamine; n is 5. | | | | | |

**Table 5**

| Effect of the PKC inhibitor or PKA inhibitor on nefiracetam-potentiated postsynaptic potential of a slice of the rat hippocampus | | | |
|---|---|---|---|
| Time (min) | Postsynaptic potential (% of original amplitude) | | |
| | GF109203X | H-89 | |
| | Mean ± SD | Mean ± SD | |
| -30 | 102 ± 3 | 103 ± 4 | |
| -10 | 100 ± 0 | 100 ± 0 | ←nef. |
| -9 | 94.2 ± 6.8 | 109 ± 2.2 | ←nef. |
| -8 | 96.2 ± 9.5 | 112 ± 4.6 | ←nef. |
| -7 | 99.7 ± 11.2 | 116 ± 6 | ←nef. |
| -6 | 99.4 ± 12.1 | 116 ± 7.2 | ←nef. |
| -5 | 105 ± 10.8 | 122 ± 9.8 | ←nef. |
| -4 | 103.4 ± 11.9 | 128 ± 11.35 | ←nef. |
| -3 | 102.5 ± 13.6 | 132 ± 17 | ←nef. |
| -2 | 101.1 ± 12.5 | 136 ± 16.3 | ←nef. |
| -1 | 108.2 ± 13.6 | 142 ± 9.8 | ←nef. |
| 0 | 106 ± 11.4 | 144 ± 8.3 | |
| 2 | 105.5 ± 11.7 | 145 ± 7.3 | |
| 4 | 99.25 ± 11 | 150 ± 7 | |
| 6 | 105.5 ± 12 | 151 ± 6.5 | |
| 8 | 104.5 ± 12.7 | 154 ± 9 | |
| 10 | 108.8 ± 11.6 | 155 ± 8 | |
| nef. ; 1 µM nefiracetam. n is 5. GF109203X ; selective inhibitor for PKC H-89 ; inhibitor for PKA (N-[2-(p-Bromocinnamylamino)ethyl]-5-isoquinolinesulfonamide · 2HCl) | | | |

**Table 6**

| Effect of nefiracetam on postsynaptic potential of a slice of the rat hippocampus (treated with paired pulses) | | |
|---|---|---|
| Interpulse Interval (sec/10000) | Facilitation (% of the 1st pulse) | |
| | Nefiracetam (-) | Nefiracetam (+) |
| | Mean ± SD | Mean ± SD |
| 50 | 164 ± 22 | 125 ± 17 |
| 100 | 134 ± 21 | 116 ± 18 |
| 150 | 124 ± 17 | 112 ± 15 |
| n = 5 | | |

Paired pulses were applied to slices at varying interpulse intervals, between 50 and 150 msec before and 10 minute after the administration of nefiracetam. The nefiracetam-administered slices showed paired-pulse facilitation of the postsynaptic potential. Briefly, reaction induced by the first pulse application was potentiated to a maximum of 120% by the next pulse application. However, the pulses were consistently lower than those obtained with slices that had not been administered nefiracetam (Table 6); therefore, nefiracetam is considered to maximally enhance release of glutamate serving as an excitatory neurotransmitter.

### Example 2 [In vitro transcription and translation in Xenopus oocytes (Tables 7 to 10; Fig. 3)]

mRNAs for the rat α4β2 receptor and α7 receptor, GLUR1, 2, 3 receptors, and NP152 and NP153 were constructed as described previously. *Xenopus* oocytes were manually separated from the ovary, and subjected to treatment with collagenase (0.5 mg/ml). Thereafter, the treated oocytes were incubated overnight in Barth's solution (in mM, 88 NaCl, 1 KCl, 2.4 NaHCO₃, 0.82 MgSO₄, 0.33 Ca(NO₂)₂, 0.41 CaCl₂, and 7.5 Tris; pH 7.6). The oocytes were injected with the α4β2 or α7 receptor mRNA and incubated at 18°C. In some cases, NP152 and NP153 were co-expressed with the α4β2 and α7 receptors. The injected oocytes were incubated and transferred to a recording chamber 2-7 days after incubation and continuously superfused at room temperature (20-22°C) with a standard frog Ringer's solution [in mM: 115 NaCl, 2 KCl, 1.8 CaCl₂, 5 HEPES; pH 7.0]. To remove the effect of endogenous muscarinic ACh receptor, 1 µM atropine was added to the extracellular solution. ACh-evoked currents were recorded by use of two-electrode voltage clamp techniques and an amplifier (GeneClamp-500, Axon Instrument, Inc. USA). The currents were analyzed on a microcomputer by pClamp software (Axon Instrument, Inc.; Version 6). From the results, an assessment was made as to whether nefiracetam actually acts on the nACh receptors. The α4β2 and α7 receptors were expressed in *Xenopus* oocytes, and current was evoked by ACh. α-Bungarotoxin was found to strongly inhibit the α7 receptor and mecamylamine was found to strongly inhibit current in the α4β2 receptor (Fig. 3).

Thus, these findings support the idea that these receptors are present in the hippocampus and participates in facilitation of the postsynaptic potential.

Furthermore, treatment with nefiracetam (1 µM) potentiated ACh-evoked currents in a time-dependent manner, reaching 180% and 195% at the time point 60 minutes after treatment in the cases of α4β2 receptor and α7 receptor, respectively (Table 7).

**Table 7**

| Effect of nefiracetam on current evoked in α7 and α4β2 receptors expressed in *Xenopus* oocytes (change over time) | | | | |
|---|---|---|---|---|
| Time (min) | % of original amplitude | | | |
| | Nefiracetam(+) | | Nefiracetam(-) | |
| | α4β2 | α7 | α4β2 | α7 |
| | Mean ± SD (n=8) | Mean ± SD (n=8) | Mean ± SD (n=5) | Mean ± SD (n=5) |
| -30 | | | 128 ± 5 | 100 ± 2 |
| -20 | | | 116 ± 4 | 100 ± 3 |
| -10 | 100 ± 0 | 100 ± 0 | 100 ± 0 | 100 ± 0 |
| 0 | 108.9 ± 4.2 | 139.3 ± 11.9 | 98 ± 3.1 | 100 ± 1 |
| 10 | 119 ± 10.6 | 155.3 ± 16 | 97 ± 4.2 | 99 ± 2 |
| 20 | 129.4 ± 13.2 | 176.6 ± 5.1 | 96 ± 3.6 | 99 ± 3 |
| 30 | 160.7 ± 12.6 | 194.6 ± 8.8 | 95 ± 5.6 | 98 ± 2 |
| 40 | 172.2 ± 14.2 | 193.6 ± 2.2 | 95 ± 2.1 | 98 ± 1 |
| 50 | 178.8 ± 12.2 | 192.1 ± 2.8 | 94 ± 3.4 | 98 ± 4 |
| 60 | 180.1 ± 9.3 | 195 ± 6.3 | 94 ± 2.2 | 97 ± 3 |

Potentiation of ACh-evoked currents was observed at concentrations of 0.0001 µM or more in the α4β2 receptor and 0.01 µM or more in the α7 receptor, reaching a maximum at concentrations of 0.1 and 1 µM, respectively (Table 8). Nefiracetam had no effect on chloride ion (Cl⁻) currents that are evoked by Ca²⁺ influx through the nACh receptor channels in oocytes (data not shown), indicating that nefiracetam acted on the nACh receptors but not on the Ca²⁺-activated chloride ion channels. ACh dose-response curve was not shifted after administration of nefiracetam into the α4β2 and α7 receptors (Table 9).

**Table 8**

| Effect of nefiracetam on α7 and α4β2 receptors expressed on the cell membrane of *Xenopus* oocytes (dose-response) | | |
|---|---|---|
| Nefiracetam (µM) | % of the original amplitude | |
| | α4β2 | α7 |
| | Mean ± SD | Mean ± SD |
| 0.0001 | 100.2 ± 15.5 | |
| 0.001 | 150.3 ± 13.6 | |
| 0.01 | 211.6 ± 20.5 | 97.4 ± 5.5 |
| 0.1 | 225.1 ± 18.9 | 181.4 ± 10.8 |
| 1 | 160.7 ± 12.6 | 194.6 ± 8.8 |
| 10 | 155.9 ± 17.6 | 116.1 ± 7.2 |
| n = 5 to 8. | | |

This implies that the potentiation of ACh-evoked currents by nefiracetam was not caused by modulation of affinity for ACh. The potentiation was fully inhibited by GF109203X in both receptors, suggesting that nefiracetam potentiated the currents by PKC activation.

In addition, nefiracetam never potentiated currents in the α4β2 and α7 receptors co-expressed with the active PKC inhibitory peptide (NP152; αPKCI)(Peunova, N. *et al.*, Nature 364, 450-453, 1993).

In contrast, currents were potentiated to 169.3% and 193.6% in the presence of the inactive PKC inhibitory peptide (NP153; iPKCI), respectively. This provides evidence that nefiracetam interacts with a PKC pathway, leading to a long-lasting potentiation of ACh-evoked currents by PKC activation.

**Table 9**

| Effect of nefiracetam treatment on Ach-evoked current in α4β2 receptors expressed on the cell membrane of *Xenopus* oocytes | | | | |
|---|---|---|---|---|
| ACh (µM) | normalized response (%) | | | |
| | α4β2 | α4β2 | α7 | α7 |
| | nef(-) Mean | nef(+) Mean | nef(-) Mean | nef(+) Mean |
| 0.1 | 0 | 0 | 0 | 0 |
| 1 | 3 | 2 | 0.5 | 1.5 |
| 10 | 43 | 36 | 1 | 3 |
| 100 | 75 | 66 | 28 | 35 |
| 1000 | 100 | 100 | 100 | 100 |
| (+)nef. ; addition of nefiracetam (1 µM) (-)nef. ; no addition of nefiracetam n = 5 | | | | |

**Table 10**

| Effects of selective PKC inhibitor and PKC inhibitory peptide on potentiation of Ach-evoked current by nefiracetam in *Xenopus* cocytes | | |
|---|---|---|
| | % of the original amplitude | |
| | α4β2 | α7 |
| | Mean ± SD | Mean ± SD |
| nef.(-) | 95 ± 5.6 | 98 ± 2 |
| nef.(+) | 160 ± 5.6 | 194.6 ± 8.8 |
| GF109203X | 79.3 ± 15 | 102.2 ± 9.6 |
| GF109203X + nef. | 80.5 ± 10 | 108 ± 6 |
| NP152 | 78 ± 12.5 | 95 ± 3.1 |
| NP152 + nef. | 78.1 ± 10 | 96.1 ± 6 |
| NP153 | 80 ± 9 | 97 ± 5 |
| NP153 + nef. | 169.3 ± 12 | 193.6 ± 17.7 |
| Staurosporine | 90 ± 8 | 101 ± 8 |
| Staurosporine + nef. | 93 ± 13 | 103 ± 12 |
| H89 | 98.8 ± 12 | 99.4 ± 5.8 |
| H89 + nef. | 163.9 ± 20 | 180.4 ± 19.6 |
| NP210 | 95.5 ± 8 | 99 ± 12 |
| NP210 + nef. | 160 ± 18 | 188 ± 21 |
| NP211 | 94 ± 11 | 98 ± 12 |
| NP211 + nef. | 162 ± 21 | 196 ± 22 |
| GDPβS | 96 ± 10 | 100 ± 11 |
| GDPβS + nef. | 185 ± 14 | 201 ± 18 |
| nef.: nefiracetam (1 µM) GF: 100 nM GF109203X; NP152: α-PKC 1, PKC-inhibitory peptide NP153: i-PKC 1, PKC-inhibitory (inactive) peptide H-89: Selective inhibitor for protein kinase A (PKA) (N-[2-p-Bromocinnamylamino)ethyl]-5-isoquinolinesulfonamide·2HCl) NP210: Active PKA-inhibitory peptide NP211: Nonactive PKA-inhibitory peptide GDPβS: Inhibitor for a broad range of G proteins n = 6 to 8. | | |

As is apparent from Table 10, significant potentiation effect of nefiracetam was observed in the presence of H-89, NP-210, or NP-211, which are associated with PKA. Similar effects were observed in the presence of an inhibitor (GDPβS) for G protein (GTP-binding protein). However, such effect of nefiracetam was never observed in the presence of Staurosporine, a selective PKC inhibitor. From all these, nefiracetam was confirmed to exert its effect by the mediation of PKC.

### Example 3 [Enhancement of glutamate release from hippocampal slices by nefiracetam]

A glutamate release test was conducted by use of guinea pig hippocampal slices in the presence or absence of tetrodotoxin (0.5 µM; TTX) and electrical stimulation (ES). The slices were treated in a standard solution containing nefiracetam (1 µM) in the presence or absence of α-bungarotoxin (50 nM) or mecamylamine (abbreviated as MCA; 3 µM) to conduct quantitative tests. Each bar indicates an average of the results from independently conducted experiments (±SD). Statistical significance was determined in accordance with ANNOVA by use of Fischer's least squares method.

The hippocampal slices were stimulated by a pair of silver electrodes (10 Hz, 5V, 0.1 msec in duration) for 10 minutes with one-minute intervals. Some slices were incubated with a standard ACSF (artificial cerebrospinal fluid) saturated with 95% O₂ and 5% CO₂ gases at 36°C in the presence or absence of tetrodotoxin. Nefiracetam was administered to the other slices in the presence or absence of α-bungarotoxin or mecamylamine. Release of glutamate into the medium was measured by HPLC.

**Table 11-1**

| Release of glutamate | |
|---|---|
| | Amount of released glutamate |
| Treatment | nmol/mg protein/10min. |
| 1 St (ES-) | 0.86 ± 0.3 |
| 2 St (ES+) | 2.03 ± 0.4 |
| 3 St (ES+)+ tetrodotoxin | 0.85 ± 0.3 |
| 4 St (ES+)+ nefiracetam | 3.23 ± 0.5 (a) |
| 5 St (ES+)+ α-bungarotoxin | 1.68 ± 0.2 (b) |
| 6 St (ES+)+ mecamylamine | 2.45 ± 0.3 (c) |

| | |
|---|---|
| (a): Significant difference of 0.01 (in comparison of (2) and (4)) | |
| (b): Significant difference of 0.01 (in comparison of (2) and (4)) | |
| (c): Significant difference of 0.1 (in comparison of (6) and (4)) | |
| St(ES-): No electrical stimulation St(ES+): Electrical stimulation | |

In the experiments using hippocampal slices, depolarization induced by electrical stimulation released excitatory neurotransmitter glutamate. Treatment with nefiracetam (1 µM) significantly increased glutamate release (Table 11-1). The increase of (release of glutamate) induced by treatment with nefiracetam was clearly inhibited by α-bungarotoxin or mecamylamine (Table 11-1), indicating that nefiracetam enhanced glutamate released by activating the neuronal nACh receptors.

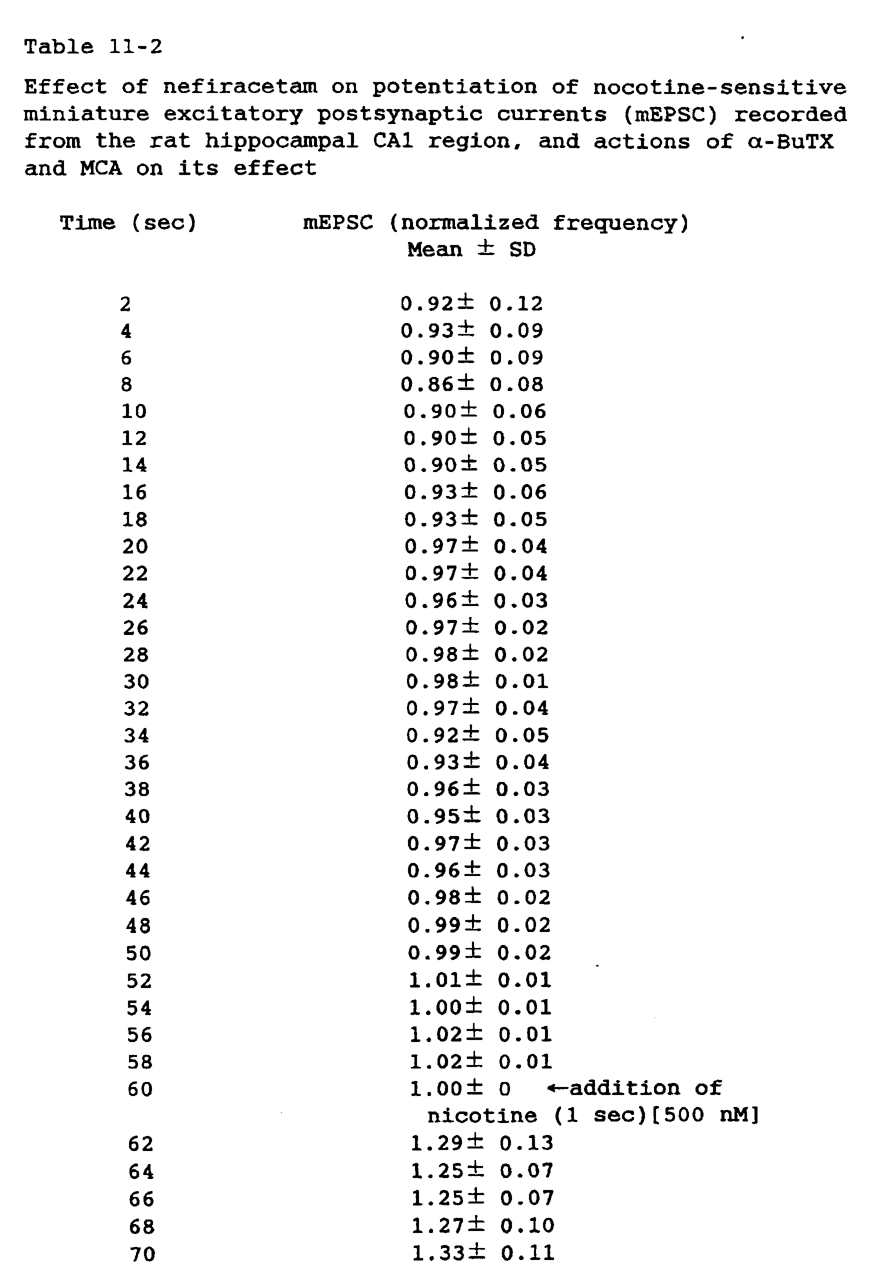

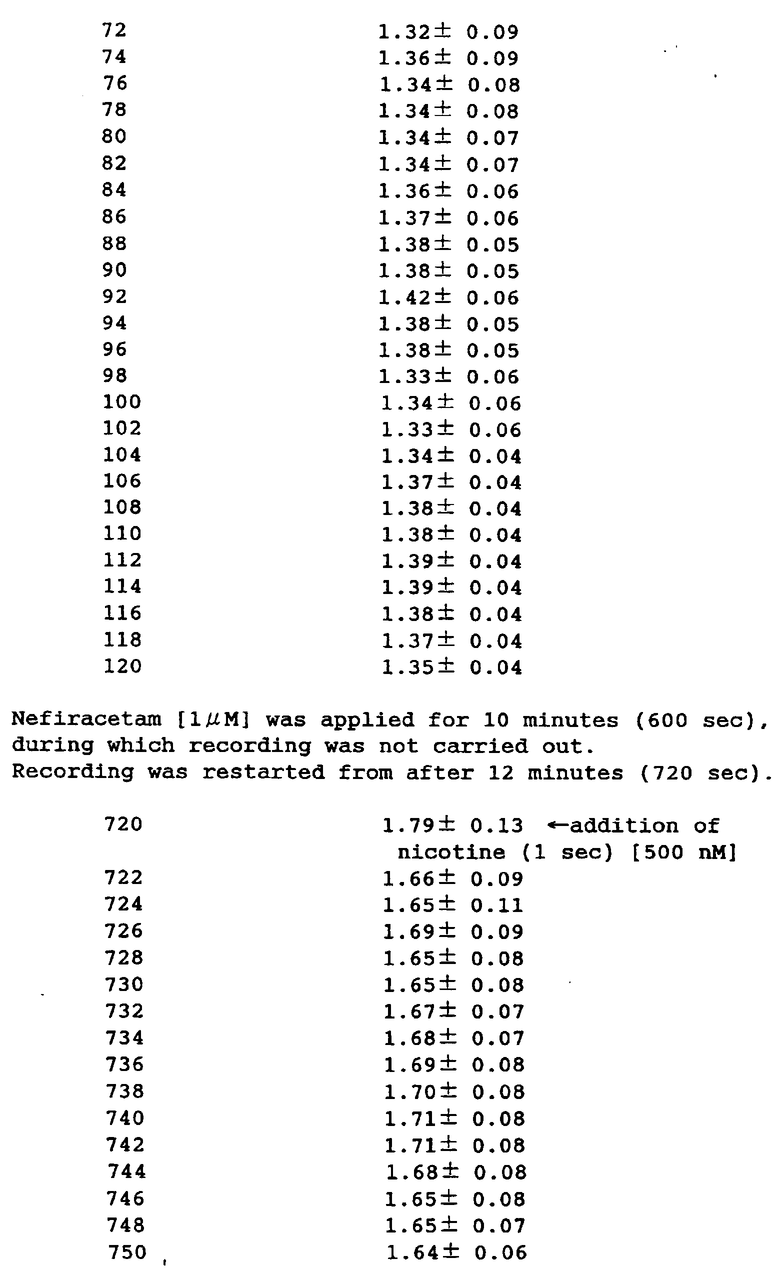

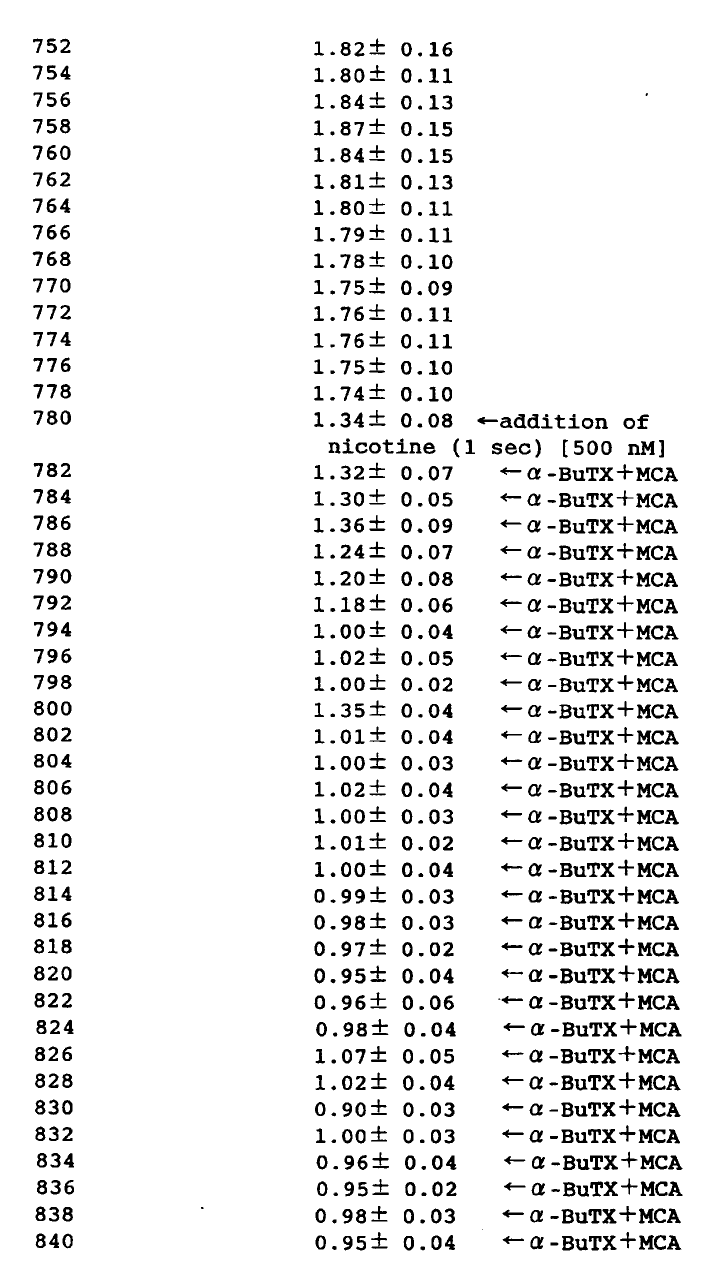

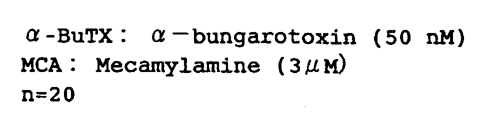

Cells were collected from the hippocampal CA1 region of a rat fetus brain The cells were cultured for 10 - 14 days, to thereby establish a primary neuron culture system. By a patch-cramp method, intrinsic nicotinic sensitive miniature excitatory postsynaptic currents (mEPSCs) of the cells were recorded. First, effect of nicotine on intrinsic mEPSCs was investigated. Potentiation attributed to nicotine was observed, which confirmed that a synapse transmission system was present in this culture system. Next, nefiracetam was added for 10 minutes. Thereafter, the present drug was sufficiently washed, and nicotine was applied. mEPSCs were further enhanced the effect exerted by nicotine alone. Subsequently, nicotine was applied in the presence of α-BuTX + MCA, which are nicotinic ACh receptor inhibitors. As a result, mEPSCs were returned to the original levels. These results suggest that the potentiation effect of nefiracetam on mEPSCs is exerted by the mediation of a nicotinic ACh receptor.

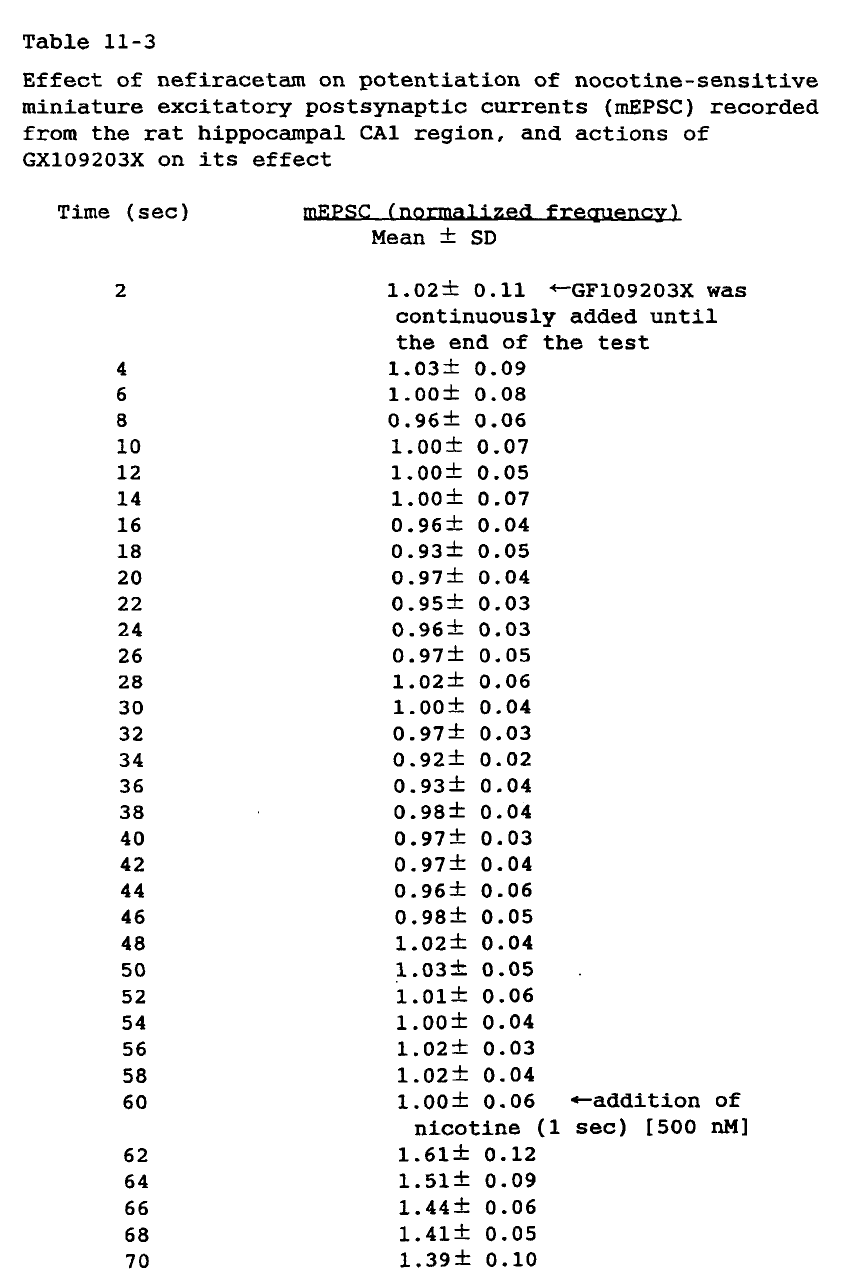

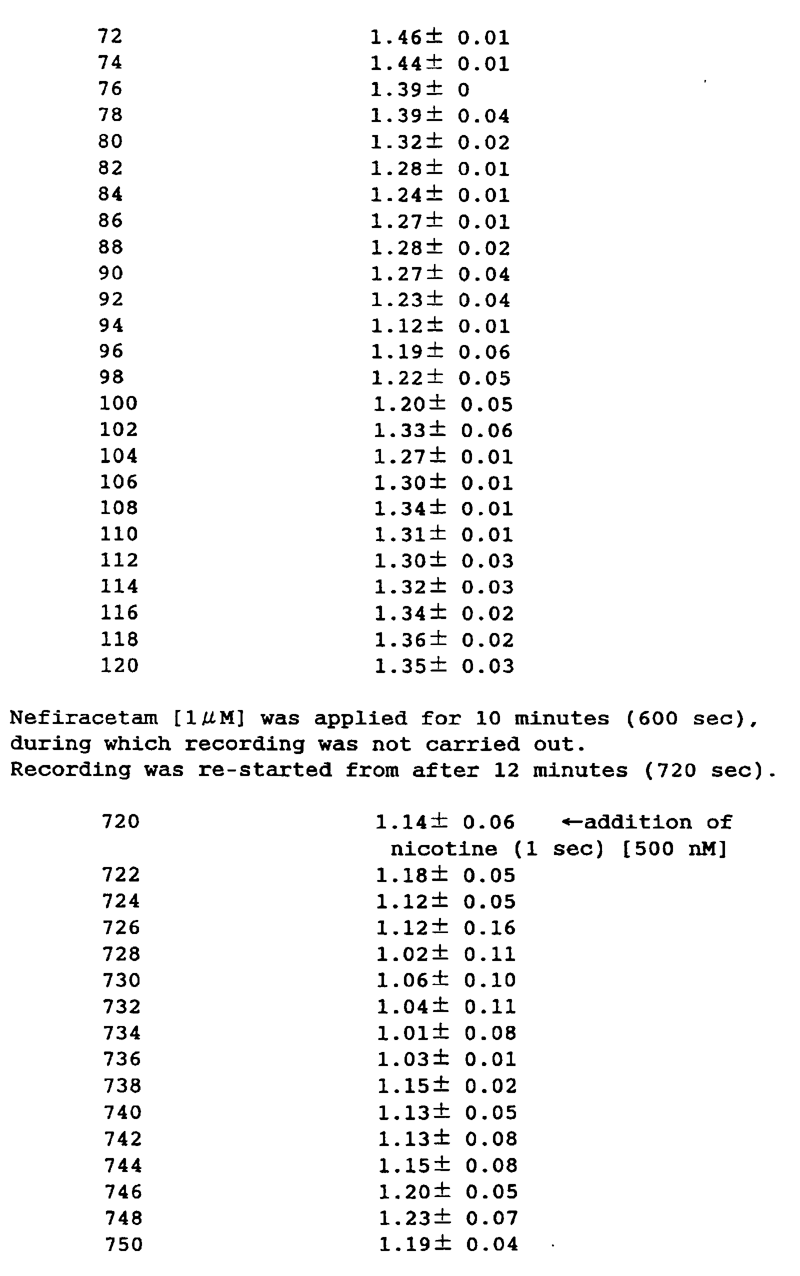

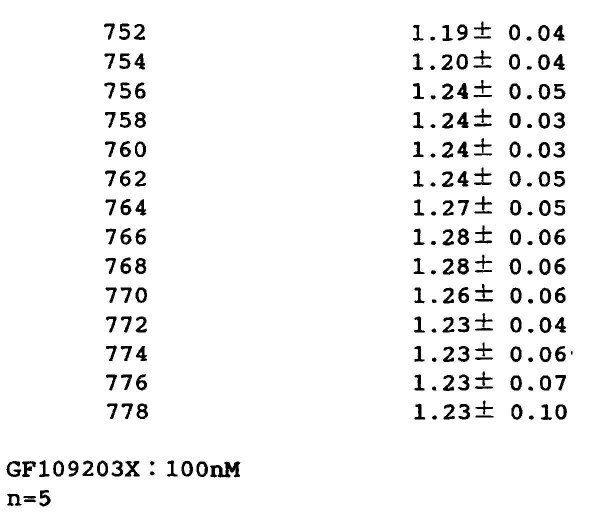

In a similar manner, and in the presence of GF109203X, a selective PKC inhibitor, effect of nicotine was first investigated. As a result, in an initial stage, nicotine potentiated intrinsic mEPSCs. Subsequently, nefiracetam was added for 10 minutes. Thereafter, the present drug was sufficiently washed, and nitotine was applied in the presence of GF109203X. As a result, mEPSCs decreased relative to the case where nicotine was used alone, and returned to the original levels. These results suggest that the potentiation effect of nefiracetam on mEPSCs is exerted by the mediation of PKC.

### Example 4a [Effect of nefiracetam on non-NMDA receptor current]

In a manner similar to that described previously, postsynaptic potentials were recorded by use of the CA1 region of the rat hippocampus. The potentials were recorded in the presence of APV (100 µM)(n = 5) or DNQX (5 µM)(n = 5) before and after administration of nefiracetam (1 µM).

**Table 12**

| Effect of nefiracetam on postsynaptic potential (non-NMDA receptor current) recorded in the rat hippocampus CA1 region. | | | |
|---|---|---|---|
| Time (min) | Postsynaptic potential (% of original amplitude) | | |
| | DNQX | APV | |
| (min) | Mean ± SD | Mean ± SD | |
| -10 | 100 ± 0 | 100 ± 0 | |
| -9 | 86 ± 6 | 95 ± 5 | |
| -8 | 81 ± 1 | 93 ± 3 | |
| -7 | 73 ± 3 | 93 ± 5 | |
| -6 | 73 ± 3 | 93 ± 3 | |
| -5 | 70 ± 9 | 89 ± 9 | |
| -4 | 62 ± 7 | 87 ± 7 | |
| -3 | 59 ± 9 | 88 ± 8 | |
| -2 | 58 ± 8 | 89 ± 11 | |
| -1 | 57 ± 7 | 88 ± 8 | |
| 0 | 59 ± 9 | 87 ± 7 | ←nef. |
| 1 | 59 ± 8 | 93 ± 9 | ←nef. |
| 2 | 63 ± 5 | 102 ± 5 | ←nef. |
| 3 | 67 ± 7 | 106 ± 6 | ←nef. |
| 4 | 76 ± 6 | 110 ± 9 | ←nef. |
| 5 | 78 ± 8 | 112 ± 8 | ←nef. |
| 6 | 78 ± 10 | 112 ± 9 | ←nef. |
| 7 | 76 ± 6 | 112 ± 7 | ←nef. |
| 8 | 77 ± 7 | 114 ± 7 | ←nef. |
| 9 | 78 ± 8 | 115 ± 8 | ←nef. |
| 10 | 79 ± 10 | 120 ± 6 | |
| nef.; nefiracetam (1µM); n = 5 APV ; D-2-amino-5-phosphonovaleric acid DNQX ; 6,7-dinitroquinoxaline-2,3-dione | | | |

Facilitation of hippocampal neurotransmission induced by nefiracetam was inhibited by 6,7-dinitroquinoxaline-2,3-dione (DNQX), a selective non-N-methyl-D-aspartate (non-NMDA) receptor antagonist (Table 12). However, it was not inhibited by D-2-amino-5-phosphonovaleric acid (APV), a selective NMDA receptor antagonist (Table 12).

The results indicate that the source for potentiation of the postsynaptic potential by nefiracetam was α-amino-3-hydroxy-5-methyl-5-methyl-4-isoxazolopropionate(AMPA)/kainate receptor currents.

### Example 4b [Effect of nefiracetam on kainate-evoked currents in GluR1, 2, 3 receptors expressed in Xenopus oocytes]

GluR1, 2, 3 receptors were expressed in *Xenopus* oocytes. Cell membrane currents evoked by kainate (100 µM) were recorded (n = 5) before and after treatment with nefiracetam (1 µM). The holding voltage was fixed at -30 mV. Nefiracetam never potentiated AMPA (GluR1, 2, 3) receptor currents (Fig. 1). Therefore, the results indicate that the potentiation of the postsynaptic potentials by nefiracetam was not caused by modulation of postsynaptic AMPA/kainate receptors.

The results presented herein clearly show that the nACh receptor acts as a target of the cognition enhancing agent.

### Example 5 [Effect of nefiracetam on ACh-evoked current]

Cell membrane currents were recorded by use of two-electrode voltage clamp techniques (T. Nishizaki *et al.*, Ikeuchi, Brain Res. 687, 214, 1995). ACh (100 µM) was administered to a single oocyte expressing normal ACh receptors at 10-min intervals before and after 10-min treatment of the oocyte with nefiracetam. The holding voltage was -30 mV. In accordance with a conventional method, wild-type acetylcholine (nACh) receptors were expressed in *Xenopus* oocytes (K. Sumikawa *et al.*, Mol. Brain Res. 5, 183, 1989), and by the application of ACh (100 µM), inward membrane currents were evoked (Table 13). Currents were recorded at 10-min intervals. During the recording period, spontaneous attenuation of the currents was within 5% (data not shown). When low (submicromolar) concentrations of nefiracetam―a nootropic agent used in the present invention―were administered for 10 min, the amplitude of the ACh-evoked currents was markedly reduced (Table 13). After the drug was washed out, the amplitude gradually recovered (Table 13). The reductions in current were 70% (n=7) with 0.01 µM and 62% with 0.1 µM nefiracetam.

**Table 13**

| Effect of nefiracetam on ACh-evoked current | | | | |
|---|---|---|---|---|
| Time (min) | % of the original amplitude Nefiracetam | | | |
| | 0.01µM | 0.1µM | 1µM | 10µM |
| | Mean ± SD | Mean ± SD | Mean ± SD | Mean ± SD |
| -10 | 100 ± 0 | 100 ± 0 | 100 ± 0 | 100 ± 0 |
| 0 | 30 ± 9 | 38 ± 11 | 134 ± 10 | 155 ± 15 |
| 10 | 37 ± 13 | 45 ± 14 | 150 ± 15 | 158 ± 11 |
| 20 | 40 ± 15 | 49 ± 15 | 155 ± 18 | 162 ± 20 |
| 30 | 63 ± 19 | 54 ± 15 | 170 ± 21 | 169 ± 16 |
| 40 | 65 ± 20 | 66 ± 16 | 188 ± 16 | 177 ± 19 |
| 50 | 75 ± 18 | 80 ± 20 | 190 ± 25 | 195 ± 20 |
| 60 | 94 ± 23 | 90 ± 21 | 209 ± 28 | 216 ± 16 |
| 70 | 104 ± 19 | 103 ± 20 | 219 ± 20 | 230 ± 18 |
| 80 | 106 ± 20 | 120 ± 19 | 244 ± 29 | 260 ± 22 |
| 90 | 105 ± 15 | 122 ± 14 | 243 ± 20 | 255 ± 18 |
| (n = 7) | | | | |

In contrast, the currents were enhanced gradually by a 10-min treatment with higher (micromolar) concentrations of nefiracetam, reaching 243% (n=7, 1 µM) or 255% (n=7, 10 µM) 90 min after washing-out of the drug (Table 13). Under conditions where currents were depressed by the administration of a submicromolar concentration (0.1 µM) of nefiracetam, micromolar application of nefiracetam (≥1 µM) caused switching from depression to enhancement (Tables 13 and 14).

This suggests that at least two different signal transduction pathways are involved in such a biphasic action of nefiracetam.

**Table 14**

| Effect of nefiracetam on nACh receptors (α, β, γ, δ) of Torpedo expressed in *Xenopus* oocytes | | |
|---|---|---|
| Time (min) | (% of the original amplitude) | |
| | Mean ± SD | |
| -10 | 100 ± 0 | ←nef. (0.01µM) |
| 0 | 32 ± 9 | |
| 10 | 39 ± 8 | |
| 20 | 43 ± 12 | ←nef. (1µM) |
| 30 | 140 ± 15 | |
| 40 | 158 ± 7 | |
| 50 | 163 ± 10 | |
| (n = 7) nef. ; nefiracetam treatment | | |

### Example 6a [Effects of nefiracetam on calcium-dependent chloride currents and calcium influx through normal nACh receptors]

Postsysnaptic potential (PS) and voltage clamp were recorded according to the above-mentioned method. ACh-receptors were expressed in *Xenopus* oocytes according to the above-mentioned method.

In atropine-free frog Ringer's solution, acetylcholine (Ach) (100 µM) was administered to an oocyte that had not expressed ACh receptors. The holding voltage was -30 mV.

In *Xenopus* oocyte expression systems, ACh-evoked currents are known to be composed of ACh-gated channel currents and calcium-dependent chloride currents (R. Miledi *et al.*, J. Physiol. 357, 173, 1984). Nefiracetam, however, had no effect on calcium-dependent chloride currents induced by stimulation from the endogenous muscarinic ACh receptors (FIG. 2).

### Example 6b

An oocyte expressing normal ACh receptors was loaded with Calcium Green. Intracellular calcium ([Ca²⁺]ᵢ) and evoked currents (IA) were recorded for each of the cases in which administration of ACh (100 µM) was carried out in a Ca²⁺-free (extCa²⁺(-)) extracellular solution or in an ordinary extracellular solution (Calcium Green TM-1 (Molecular Probes, Inc. USA) was injected to the oocytes). The Ca²⁺ rise was normalized by Δ increase (δI) of intensity/base intensity/current amplitude (µA) obtained with calcium-free media.

In Table 15, each value represents the average from seven oocytes (SD; standard deviation). ACh-evoked currents in the *Xenopus* oocytes expression system are composed of acetylcholine-gated channel currents and calcium-dependent chloride currents. Nefiracetam had no effect on calcium permeability through the nACh receptor channel. (Table 15). The results from Examples 6a and 6b indicate that nefiracetam modulated acetylcholine-gated channel currents but not calcium-sensitive chloride (Cl⁻) currents.

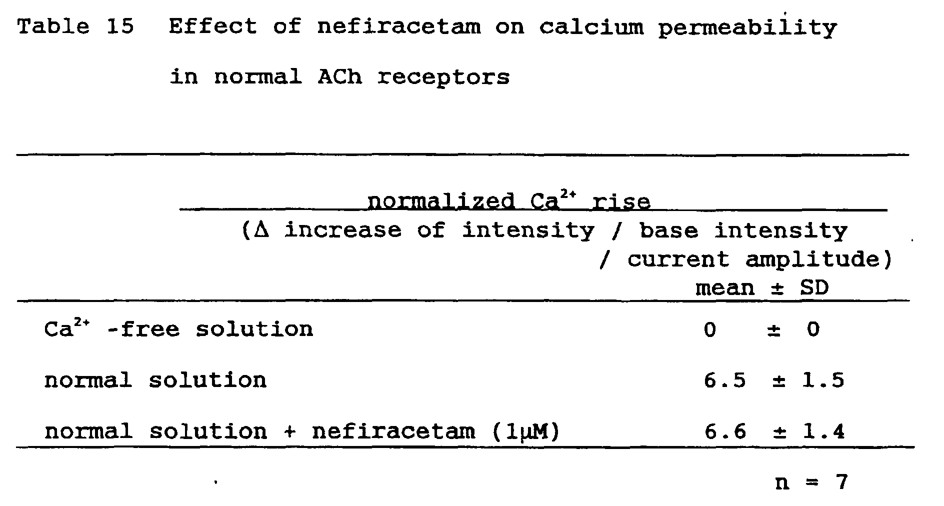

### Example 7a [Regulation of ACh-evoked currents by nefiracetam-mediated PKA activation]

ACh (100 µM) was applied to each of an oocyte expressing normal ACh receptors and an oocyte expressing mutant ACh receptors (mγΔPKA/Ser353, 354m δΔPKA/Ser361,362) lacking PKA phosphorylation sites, before and after treatment with nefiracetam (0.01 µM). The oocytes expressing normal ACh receptors were treated with H-89 (1 µM) from 15 min before recording, or with pertussis toxin (0.1 µg/ml) for 24h prior to experiments. The currents illustrated in Table 16 were recorded before and 30 min after treatment with nefiracetam. The holding voltage was - 30 mV. In Table 16, each point represents the average obtained from seven oocytes.

An attempt has been made to elucidate the intracellular signalings that mediate the nefiracetam-induced current depression and potentiation.

H89, a selective inhibitor of cAMP-dependent protein kinase (PKA), inhibited the inhibitory action of 0.01 µM nefiracetam, but conversely, potentiated the currents. The amount of potentiation was 72% (n=7) 30 min after treatment with nefiracetam (Table 16). This suggests that at lower concentrations nefiracetam caused depression of currents via PKA activation.

Likewise, nefiracetam (0.01 µM) inhibited the inhibitory action of ACh-evoked currents in an experiment in which oocytes treated with pertussis toxin (PTX), a G-protein (Gi/o) inhibitor, to the same level as that observed in the case of H89 treatment, but, conversely, potentiated the currents (Table 16).

This suggests that the inhibitory action of nefiracetam was mediated by PKA activation via pertussis toxin-sensitive G-proteins.

In addition, the facilitatory action of nefiracetam at micromolar (µM) concentrations was more enhanced in the presence of H89 (data not shown). The amount of increase was 136% (n=5) when observed 30 min after treatment with 1 µM nefiracetam in the presence of H89 (data not shown). This strongly suggests that higher concentrations (≥ 1 µM) of nefiracetam can still activate PKA and maintain the current-depression effects.

However, such inhibitory effects will be masked by the apparent current potentiation.

To further examine whether the current depression by nefiracetam is due to PKA phosphorylation of the receptors or not, mutant ACh receptors lacking potent PKA phosphorylation sites on the γ and δ subunits (mγΔ PKA/Ser353,354m δΔPKA/Ser361,362) were expressed in *Xenopus* oocytes.

There, nefiracetam (0.01 µM) did not decrease ACh-evoked currents in the mutant ACh receptors, but, conversely, increased them to 159% (n=7) when measured 30 min after treatment (Table 16). This indicates that nefiracetam inhibited the currents as a result of PKA-activation-induced PKA phosphorylation of the ACh receptors.

To further examine whether or not the current depression is due to PKA phosphorylation of the nACh receptors, mutant ACh receptors lacking potent PKA phosphorylation sites on the γ and δ subunits (mγΔ PKA/Ser353,354m δΔPKA/Ser361,362) were expressed in the cell membrane of *Xenopus* oocytes. The substance of the present invention (0.01 µM) did not reduce ACh-evoked currents in the mutant ACh receptors, but, conversely, increased them (Table 16). This indicates that the substance of the present invention inhibited the currents by PKA phosphorylation on the ACh receptors attributable to PKA activation.

It is clearly shown that the substance of the present invention affects two types of signal transmission pathways as demonstrated by potentiation of ACh-gated channel currents and activation of PKA based on the mechanisms; i.e., activation of calcium-dependent PKC and phosphorylation of ACh receptors by PKC.

**Table 16**

| Regulation of ACh-evoked currents caused by PKA-activation by the mediation of nefiracetam | | | | |
|---|---|---|---|---|
| | % of the original amplitude | | | |
| Time (min) | αβγδ | αβγδ+H89 | αβγδ+PTX | m γ Δ PKA |
| | | | | m δ Δ PKA |
| | Mean ± SD | Mean ± SD | Mean ± SD | Mean ± SD |
| -10 | 100 ± 0 | 100 ± 0 | 100 ± 0 | 100 ± 0←nef. |
| 0 | 30 ± 9 | 166 ± 16 | 154 ± 4 | 115 ± 4 |
| 10 | 37 ± 13 | 170 ± 20 | 156 ± 10 | 118 ± 6 |
| 20 | 40 ± 15 | 173 ± 18 | 160 ± 7 | 151 ± 6 |
| 30 | 63 ± 19 | 172 ± 13 | 168 ± 11 | 159 ± 20 |
| nef. ; nefiracetam (0.01 µM) treatment. (n=7) | | | | |

### Example 7b [Effects of nefiracetam on oocytes expressing normal ACh receptors or mutant ACh receptors lacking PKA phosphorylation sites]

ACh (100 µM) was applied to each of an oocyte expressing normal ACh receptors and an oocyte expressing mutant ACh receptors (mγΔPKA/Ser353,354m δΔPKA/Ser361,362) lacking PKA phosphorylation sites, in the presence and absence of GF109203X (100 nM) or in calcium-free extracellular solution.

Each value represents the average obtained from seven oocytes. The potentiation of ACh-evoked currents by different micromolar concentrations (1 to 10 µM) of nefiracetam was inhibited and reducd in the presence of GF109203X, a selective PKC inhibitor; i.e., to 60% (n=7) when 30 minutes has elapsed after administration of nefiracetam (Table 17). This indicates that nefiracetam potentiated ACh receptor currents via PKC activation. In calcium-free media, the potentiation was never observed, and the currents were reduced to an extent similar to that with GF109203X (Table 17).

Furthermore, nefiracetam (1 µM) exhibited no current potentiation in the mutant ACh receptors that lack potent PKC phosphorylation sites on the α and δ subunits (mαγΔ PKC/Ser333mδ ΔPKC/Ser377)(V. M. Gehle *et al.*, Mol. Brain Res. 5. 183, 1991)(Table 17). These results indicate that nefiracetam potentiated ACh-gated channel currents by activation of calcium-dependent PKC and the following PKC phosphorylation of the receptors. Nefiracetam thus appears to act on two different signal transduction pathways; one is responsible for pertussis toxin-sensitive G-protein-regulated PKA activation and the other for calcium-dependent PKC activation.

**Table 17**

| Regulation of ACh-evoked currents caused by PKC activation by the mediation of nefiracetam | | | | |
|---|---|---|---|---|
| | % of the original amplitude | | | |
| Time (min) | αβγδ | mαΔPKC mδΔPKC | αβγδ+ GF109203X | αβγδ+ extCa²⁺(-) |
| | Mean ± SD | Mean ± SD | Mean ± SD | Mean ± SD |
| -10 | 100 ± 0 | 100 ± 0 | 100 ± 0 | 100 ± 0←nef. |
| 0 | 134 ± 10 | 74 ± 3 | 70 ± 3 | 81 ± 4 |
| 10 | 150 ± 15 | 75 ± 7 | 68 ± 15 | 61 ± 6 |
| 20 | 155 ± 18 | 59 ± 7 | 63 ± 9 | 52 ± 7 |
| 30 | 170 ± 21 | 50 ± 6 | 60 ± 16 | 48 ± 5 |
| nef. ; treated with nefiracetam (1µM) (n = 7) | | | | |

### Example 8A [Effects of nefiracetam on postsynaptic potential (PS)]

According to the above-mentioned method, postsynaptic potentials were recorded from the CA1 region of a pyramidal cell layer. Hippocampal slices were prepared from the rat brain and the field postsynaptic potentials were recorded from the CA1 region of the pyramidal cell layer. The slices were treated with nefiracetam at concentrations as indicated. The time course effects of nefiracetam at each concentration are summarized in Table 18. Each value represents % of the postsynapstic potential recorded at -10 min (baseline)(n=6). To assess a functional role in nefiracetam-mediated signaling, the dendritic field postsynaptic potentials were recorded from the CA1 region of the pyramidal cell layer.

Interestingly, nefiracetam here also exerted a dose-dependent biphasic effect on the postsynaptic potentials; a short-term suppression at lower concentrations (≤ 0.1 µM) and a long-lasting enhancement similar to that of LTP at higher concentrations (≥ 1 µM) (Table 18-1).

**Table 18-1**

| Effect of nefiracetam on postsynaptic potential (PS) | | | | |
|---|---|---|---|---|
| Time (min) | Postsynaptic potential(PS)(% of original amplitude) Nefiracetam | | | |
| | 0.01µM | 0.1µM | 1µM | 10µM |
| | Mean ± SD | Mean ± SD | Mean ± SD | Mean ± SD |
| -30 | 100 ± 2 | 100 ± 5 | 100 ± 3 | 100 ± 4 |
| -20 | 100 ± 5 | 100 ± 3 | 100 ± 7 | 100 ± 5 |
| -10 | 100 ± 0 | 100 ± 0 | 100 ± 0 | 100 ± 0←nef. |
| -9 | 95 ± 5 | 100 ± 8 | 108 ± 1 | 97 ± 7←nef. |
| -8 | 90 ± 9 | 96 ± 9 | 109 ± 2 | 110 ± 6←nef. |
| -7 | 81 ± 9 | 91 ± 9 | 111 ± 2 | 115 ± 6←nef. |
| -6 | 75 ± 8 | 87 ± 6 | 122 ± 8 | 121 ± 8←nef. |
| -5 | 76 ± 8 | 85 ± 9 | 133 ± 6 | 116 ± 3←nef. |
| -4 | 74 ± 8 | 81 ± 13 | 127 ± 4 | 122 ± 9←nef. |
| -3 | 75 ± 6 | 75 ± 6 | 132 ± 7 | 126 ± 7←nef. |
| -2 | 74 ± 7 | 75 ± 9 | 132 ± 7 | 138 ± 7←nef. |
| -1 | 79 ± 4 | 78 ± 10 | 131 ± 9 | 135 ± 6←nef. |
| 0 | 81 ± 11 | 81 ± 6 | 131 ± 8 | 133 ± 8 |
| 2 | 80 ± 7 | 81 ± 5 | 136 ± 9 | 135 ± 4 |
| 4 | 81 ± 8 | 83 ± 5 | 129 ± 6 | 135 ± 6 |
| 6 | 83 ± 12 | 85 ± 9 | 131 ± 9 | 135 ± 8 |
| 8 | 85 ± 9 | 87 ± 13 | 135 ± 9 | 137 ± 6 |
| 10 | 85 ± 5 | 87 ± 12 | 143 ± 2 | 137 ± 6 |
| 15 | 86 ± 5 | 88 ± 9 | 144 ± 1 | 143 ± 6 |
| 20 | 85 ± 9 | 94 ± 10 | 142 ± 12 | 144 ± 2 |
| 25 | 85 ± 13 | 100 ± 14 | 144 ± 8 | 143 ± 5 |
| 30 | 86 ± 5 | 101 ± 7 | 140 ± 10 | 143 ± 7 |
| 35 | 86 ± 1 | 106 ± 8 | 141 ± 10 | 144 ± 8 |
| 40 | 89 ± 3 | 111 ± 10 | 155 ± 5 | 145 ± 12 |
| 45 | 88 ± 4 | 109 ± 9 | 153 ± 9 | 142 ± 5 |
| 50 | 92 ± 2 | 111 ± 6 | 155 ± 10 | 142 ± 5 |
| 55 | 93 ± 6 | 110 ± 10 | 156 ± 7 | 143 ± 9 |
| 60 | 94 ± 5 | 111 ± 9 | 156 ± 6 | 144 ± 11 |
| (n = 6) | | | | |

**Table 18-2**

| Effect of nefiracetam on field excitatory postsynaptic potential (fEPSP) | | |
|---|---|---|
| | fEPSP(% of baseline EPSP slope) | |
| Time (min) | Control | Nefiracetam (1 µM) |
| | Mean ± SD | Mean ± SD |
| -30 | 102± 3 | 103 ± 5 |
| -10 | 100± 0 | 100 ± 0 ←addition of nefiracetam (10min) |
| 0 | 101± 2 | 120 ± 8 |
| 5 | 100± 4 | 151 ± 10 |
| 10 | 99± 5 | 173 ± 18 |
| 15 | 98± 2 | 170 ± 8 |
| 20 | 97± 4 | 175 ± 14 |
| 25 | 100± 3 | 180 ± 12 |
| 30 | 95± 2 | 180 ± 15 |
| 35 | 98± 6 | 182 ± 17 |
| 40 | 95± 5 | 183 ± 16 |
| 45 | 92± 4 | 185 ± 19 |
| 50 | 99± 3 | 190 ± 17 |
| 55 | 97± 6 | 195 ± 14 |
| 60 | 95± 8 | 192 ± 16 |
| 90 | 94± 15 | 191 ± 18 |
| 120 | 96± 14 | 198 ± 20 |
| 150 | 97± 9 | 198 ± 12 |
| 180 | 92± 17 | 190 ± 19 |
| 210 | 91± 14 | 186 ± 14 |
| 240 | 90± 12 | 180 ± 20 |
| Control group: n=5 Nefiracetam group: n=7 | | |

Long-lasting facilitation of hippocampal synaptic transmission induced by nefiracetam was investigated. The field excitatory postsynaptic potentials (fEPSP) were recorded in the pyramidal cell layer of the CA1 region by electrical stimulation to the Schaeffer collateral pathway in rat hippocampla slices. Nefiracetam (1 µM) was applied to the hippocampla slices for 10 minutes. After nefiracetam application, fEPSPs were gradually augmented. This effect was observed until 240 minutes had passed since the experiment ended (Table 18-2). On the other hand, no change was observed in the control group (non-treatment group). These results demonstrate that nefiracetam facilitates fEPSPs for a prolonged period without tatanic stimulation.

Next, investigation was made regarding whether the above effect exerted by the present drug differs from the onset mechanism for long-term potentiation (LTP) action exerted when tetanic stimulation was used. The following three groups were compared: (group 1) tetanic stimulation alone; (group 2) nefiracetam + tetanic stimulation; (group 3) tetanic stimulation + nefiracetam. As a result, the fEPSPs that had been potentiated by nefiracetam was not potentiated any longer even when subsequent tetanic stimulation was applied. Likewise, the fEPSPs that had been potentiated by tetanic stimulation was not potentiated any longer even after subsequent application of nefiracetam. The amplitudes of potentiation of these two types of fEPSP were the same as the amplitude of tetanic stimulation. From these results, the action of nefiracetam was suggested to have a mechanism shared by LTP (Table 18-3).

**Table 18-3**

| Comparison regarding interactions between tetanic stimulation and nefiracetam on field excitatory postsynaptic potential | | | |
|---|---|---|---|
| | fEPSP(% of baseline EPSP slope) | | |
| Time (min) | Tetamic stimulation alone | Nefiracetam + tetanus stimulation | Tetanus stimulation + nefiracetam |
| | Mean ± SD | Mean ± SD | Mean ± SD |
| -30 | 102± 3 | 103 ± 5 | 102 ± 4 |
| -10 | 100± 0 | 100 ± 0 ←nef. | 100 ± 0 |
| 0 | 101± 6←tetanus | 119 ± 15 | 102 ± 20←tetanus |
| 5 | 195± 19 | 155 ± 15 | 197 ± 22 |
| 10 | 184± 18 | 173 ± 11 | 187 ± 24 |
| 15 | 190± 14 | 181 ± 18 | 189 ± 25 |
| 20 | 184± 26 | 182 ± 12 | 195 ± 27 |
| 25 | 182± 24 | 188 ± 16 | 199 ± 25 |
| 30 | 189± 22 | 187 ± 23 | 192 ± 24 |
| 35 | 193± 18 | 189 ± 25 | 192 ± 23 |
| 40 | 194± 21 | 182 ± 26 | 187 ± 24 |
| 45 | 188± 22 | 184 ± 19 | 193 ± 24 |
| 50 | 193± 19 | 186 ± 21 | 197 ± 25 |
| 55 | 195± 23 | 182 ± 20 | 188 ± 29 |
| 60 | 196± 24 | 185 ± 18←tetanus | 191 ± 24←nef. |
| 65 | 201± 26 | 198 ± 18 | 191 ± 17 |
| 70 | 194± 22 | 178 ± 21 | 197 ± 26 |
| 75 | 190± 19 | 183 ± 18 | 203 ± 32 |
| 80 | 188± 22 | 191 ± 20 | 213 ± 33 |
| 85 | 195± 28 | 185 ± 16 | 208 ± 21 |
| 90 | 199± 24 | 189 ± 24 | 206 ± 30 |
| 95 | 196± 21 | 187 ± 22 | 206 ± 34 |
| 100 | 194± 18 | 192 ± 19 | 207 ± 31 |
| 105 | 200± 25 | 194 ± 26 | 201 ± 31 |
| 110 | 201± 21 | 192 ± 13 | 201 ± 32 |
| 115 | 198± 27 | 191 ± 13 | 209 ± 30 |
| 120 | 197± 19 | 174 ± 22 | 208 ± 33 |
| tetanus: tetanic stimulation (100 Hz for 1 sec) nef.: addition of nefiracetam (for 10 minutes) tetanic stimulation: n=7 nefiracetam + tetanic stimulation: n=7 tetanic stimulation + nefiracetam: n=7 | | | |

Since LTP is the cellular model for memory most intensively studied (T.V.P. Bliss *et al.*, Nature 361, 31, 1993), such an LTP-like effect observed here may be a common mechanism by which nootropic agents improve memory and learning (M. Sarter *et al.*, Trends Pharmacol. Sci. 12, 456, 1991).

### Example 8b [Effects of nefiracetam on postsynaptic potential in the presence of α-bungarotoxin or mecamylamine]

In the presence of α-bungarotoxin (100 nM)(n=5) or mecamylamine (3 µM)(n=5), nefiracetam (1 µM) was administered to a hippocampal slice. A long-lasting enhancement of postsynaptic potential by nefiracetam was inhibited in the presence of α-bungarotoxin, which is a selective α7 nACh receptor antagonist, or mecamylamine, which is a non-selective neuronal nACh receptor antagonist (Table 19-1).

**Table 19-1**

| Effect of nefiracetam on postsynaptic potential in the presence of α-bungarotoxin or mecamylamine | | | |
|---|---|---|---|
| Time (min) | Postsynaptic potential (% of original amplitude) | | |
| | α-BuTX | mecamylamine (3µM) | |
| | Mean ± SD | Mean ± SD | |
| -30 | | 100 ± 5 | |
| -10 | 100 ± 4 | | |
| -5 | 100 ± 0 | 100 ± 0 | ←BuTX or meca. |
| -4 | 77 ± 6 | 81 ± 6 | ←BuTX or meca. |
| -3 | 63 ± 5 | 56 ± 5 | ←BuTX or meca. |
| -2 | 43 ± 1 | 50 ± 9 | ←BuTX or meca. |
| -1 | 44 ± 2 | 47 ± 4 | ←BuTX or meca. |
| 0 | 43 ± 1 | 41 ± 3 | ←BuTX or meca. + nefi. |
| 1 | 52 ± 2 | 63 ± 7 | ←BuTX or meca. + nefi. |
| 2 | 54 ± 9 | 63 ± 5 | ←BuTX or meca. + nefi. |
| 3 | 53 ± 9 | 63 ± 4 | ←BuTX or meca. + nefi. |
| 4 | 54 ± 5 | 63 ± 8 | ←BuTX or meca. + nefi. |
| 5 | 53 ± 10 | 65 ± 10 | ←BuTX or meca. + nefi. |
| 6 | 55 ± 4 | 68 ± 5 | ←BuTX or meca. + nefi. |
| 7 | 55 ± 6 | 70 ± 4 | ←BuTX or meca. + nefi. |
| 8 | 59 ± 8 | 73 ± 7 | ←BuTX or meca. + nefi. |
| 9 | 64 ± 3 | 75 ± 6 | ←BuTX or meca. + nefi. |
| 10 | 65 ± 7 | 78 ± 9 | |
| 11 | 63 ± 3 | 95 ± 10 | |
| 12 | 63 ± 7 | 97 ± 8 | |
| 13 | 65 ± 6 | 108 ± 7 | |
| 14 | 75 ± 3 | 122 ± 8 | |
| 15 | 83 ± 8 | 126 ± 5 | |
| 16 | 133 ± 5 | 134 ± 5 | |
| 17 | 133 ± 9 | 145 ± 10 | |
| 18 | 133 ± 4 | 150 ± 9 | |
| 19 | 140 ± 5 | 153 ± 8 | |
| 20 | 141 ± 8 | 153 ± 6 | |
| BuTX ; α-bungarotoxin (100 µM) meca ; mecamylamine (3 µM) nefi. ; Nefiracetam (1 µM) (n = 5) | | | |

**Table 19-2**

| Action of α-BuTX on the effect of nefiracetam regarding field excitatory postsynaptic potentials (fEPSPs) | |
|---|---|
| | fEPSP(% of baseline EPSP slope) |
| Time (min) | Nefiracetam (1 µM) |
| | Mean ± SD |
| -30 | 103± 1.5 |
| -20 | 96± 6 ←Continuous addition of α-BuTX from this point until the end of the experiment |
| -15 | 98± 10.5 |
| -10 | 99± 7.5 ←addition of nefiracetam (10min) |
| -5 | 98± 6 |
| 0 | 99± 10.5 |
| 5 | 92± 4.5 |
| 10 | 105± 5.5 |
| 15 | 100± 5.5 |
| 20 | 104± 3 |
| 25 | 106± 6.7 |
| 30 | 108± 8 |
| 35 | 108± 6.5 |
| 40 | 102± 5.3 |
| 45 | 104± 7.3 |
| 50 | 108± 4.5 |
| 55 | 102± 5 |
| 60 | 106± 5.5 |
| n=5 α-BuTX : 50 nM | |

In a manner similar to that described above, and by use of hippocampal slices, effect of nefiracetam on field excitatory postsynaptic potentials (fEPSPs) was investigated in the presence of a nicotinic receptor inhibitor. Two types of neuronal nicotinic ACh receptors, i.e., α7 and α4β2 receptors, have been known. When nefiracetam was applied in the presence of α-BuTX (selective α7 inhibitor) and MCA (selective α4β2 inhibitor), its potentiation effect was never observed at all (Tables 19-2 and 19-3). From this, the augmentation effect of nefiracetam was suggested to be exerted by the mediation of nicotinic ACh receptors.

**Table 19-3**

| Effect of MCA on nefiracetam-induced potentiation of field excitatory postsynaptic potentials (fEPSPs) | |
|---|---|
| | fEPSP(% of baseline EPSP slope) |
| Time (min) | Nefiracetam (1 µM) |
| | Mean ± SD |
| -30 | 103± 3 |
| -20 | 95± 2 ←Continuous addition of MCA from this point until the end of the experiment |
| -15 | 97± 4 |
| -10 | 100± 0 ←addition of nefiracetam (10min) |
| -5 | 98± 2.5 |
| 0 | 102± 6.5 |
| 5 | 96± 6.5 |
| 10 | 91± 4 |
| 15 | 94± 3 |
| 20 | 96± 2.5 |
| 25 | 93± 4 |
| 30 | 94± 6 |
| 35 | 97± 1.5 |
| 40 | 100± 5 |
| 45 | 98± 2 |
| 50 | 99± 3.5 |
| 55 | 101± 5.5 |
| 60 | 103± 8 |
| n=5 MCA: 3µM | |

### Example 8c [Effects of nefiracetam on postsynaptic potential in the presence of PKC inhibitor or PKA inhibitor]

The postsynaptic potentials were recorded before and after treatment with nefiracetam (0.01 and 1 µM) in the presence or absence of H-89 (1 µM) or GF109203X (100 nM). Each value represents % of baseline at 10 min after treatment with nefiracetam (n=5). As in the case of the oocyte expression system, suppression at a low concentration of nefiracetam (0.1 µM) was inhibited by treatment with H89, and potentiation at a high concentration of nefiracetam was inhibited by GF109203X (Table 20).

This indicates that nefiracetam modulated hippocampal neurotransmission differentially via PKA and PKC activation.

**Table 20**

| Effect of nefiracetam treatment on hippocampal postsynaptic potential in the presence of H89 or GF109203X Postsynaptic potential (% of original amplitude) | | | |
|---|---|---|---|
| Nefiracetam (µM) | Control | H-89(1µM) | GF109203X(100nM) |
| | Mean ± SD | Mean ± SD | Mean ± SD |
| 0.01 | 85 ± 5 | 103 ± 8 | 50 ± 5 |
| 1 | 143 ± 2 | 155 ± 8 | 108.8 ± 11.6 |

Although the data presented in the ACh-evoked current study was obtained from Torpedo nACh receptors, micromolar concentrations of nefiracetam induced a long-lasting potentiation of currents by PKC activation also in cells in which α7 and α4 β2 receptors―which are present most abundantly as neuronal nACh receptors―had been expressed (P.B.S. Clarke *et al.*, J. Neurosci. 5, 1307, 1985: E. Wadabe *et al.*, J. Comp. Neurol. 284, 314, 1989: C. M. Flores *et al.*, Mol. Pharmacol. 41, 31, 1992: E. Dominguez del Toro *et al.*, J. Comp. Neurol. 349, 325, 1994). This suggests that the action of nefiracetam on Torpedo nACh receptors can be generalized to that on the neuronal nACh receptors.

**Table 21**

| Effects of H-89 on nefiracetam-induced potentiation of field excitatory postsynaptic potentials (fEPSPs) | |
|---|---|
| | fEPSP(% of baseline EPSP slope) |
| Time (min) | Nefiracetam (1 µM) |
| | Mean ± SD |
| -30 | 108± 3 |
| -20 | 103± 4 ←Continuous addition of H-89 from this point until the end of the experiment |
| -15 | 105± 5 |
| -10 | 100± 0 ←addition of nefiracetam (10min) |
| -5 | 114± 17 |
| 0 | 124± 20 |
| 5 | 149± 21 |
| 10 | 158± 22 |
| 15 | 164± 21 |
| 20 | 170± 22 |
| 25 | 179± 24 |
| 30 | 175± 23 |
| 35 | 185± 24 |
| 40 | 180± 21 |
| 45 | 182± 22 |
| 50 | 178± 21 |
| 55 | 180± 22 |
| 60 | 184± 18 |
| n=5 H-89 : 1µM H-89 : N-2-(p-Bromocinnamylamino)ethyl] -5-isoquinolinesulfonamide · 2HCl | |

In a manner similar to that described above, and by use of hippocampal slices, effect of nefiracetam on field excitatory postsynaptic potentials (fEPSPs) was investigated in the presence of a PKC inhibitor or PKA inhibitor. When nefiracetam was applied in the presence of H-89 (selective inhibitor for PKA), significant potentiation effect was observed, while when nefiracetam was applied in the presence of GF109203X (selective inhibitor for PKC), potentiation effect was never observed (Tables 21 and 22). From this, the augmentation effect attributable to nefiracetam was suggested to be exerted by the mediation of PKC.

**Table 22**

| Effects of GF109203X on nefiracetam-induced potentiation of field excitatory postsynaptic potentials (fEPSPs) | |
|---|---|
| | fEPSP(% of baseline EPSP slope) |
| Time (min) | Nefiracetam (1 µM) |
| | Mean ± SD |
| -30 | 102± 3 |
| -20 | 100± 4 ←Continuous addition of GF109203X from this point until the end of the experiment |
| -15 | 98± 5 |
| -10 | 100± 0 ←addition of nefiracetam (10min) |
| -5 | 98± 7 |
| 0 | 96± 10 |
| 5 | 99± 11 |
| 10 | 99± 12 |
| 15 | 105± 11 |
| 20 | 103± 12 |
| 25 | 103± 14 |
| 30 | 101± 13 |
| 35 | 108± 14 |
| 40 | 106± 11 |
| 45 | 106± 12 |
| 50 | 99± 11 |
| 55 | 106± 12 |
| 60 | 105± 13 |
| n=5 GF109203X : 100nM | |

**Table 23**

| Effects of APV on nefiracetam-induced potentiation of field excitatory postsynaptic potentials (fEPSPs) | |
|---|---|
| | fEPSP(% of baseline EPSP slope) |
| Time (min) | Nefiracetam (1 µM) |
| | Mean ± SD |
| -30 | 118± 11 |
| -20 | 105± 12 ←Continuous addition of APV from this point until the end of the experiment |
| -15 | 102± 9 |
| -10 | 100± 0 ←addition of nefiracetam (10min) |
| -5 | 105± 12 |
| 0 | 102± 9 |
| 5 | 135± 9 |
| 10 | 148± 10 |
| 15 | 172± 11 |
| 20 | 184± 6 |
| 25 | 194± 12 |
| 30 | 189± 16 |
| 35 | 194± 13 |
| 40 | 187± 11 |
| 45 | 193± 14 |
| 50 | 196± 9 |
| 55 | 199± 10 |
| 60 | 196± 11 |
| n=5 APV : 100µM APV : 2-amino-5-phosphonovalerate | |

In a manner similar to that described above and by use of hippocampal slices, effect of nefiracetam on field excitatory postsynaptic potentials (fEPSPs) was investigated in the presence of an NMDA receptor inhibitor. When nefiracetam was applied in the presence of APV (selective inhibitor for NMDA), significant potentiation effect was observed (Table 23). This suggests that the augmentation effect attributable to nefiracetam is exerted without mediation of an NMDA receptor.

**Table 24**

| Effect of nefiracetam on excitatory postsynaptic potential in the brain (*in vivo*) | | |
|---|---|---|
| | Excitatory postsynaptic potential (% of baseline spike amplitude) | |
| Time (min) | Control | Nefiracetam (7.5mg/kg) |
| | Mean ± SD | Mean ± SD |
| -30 | 98± 6 | 99± 5 |
| -20 | 102± 5 | 105 ± 3 |
| -10 | 100± 4 | 98 ± 3 |
| 0 | 100± 0 | 100 ± 0 ←administration of nefiracetam |
| 10 | 103± 4 | 120 ± 3 |
| 20 | 100± 5 | 127 ± 9 |
| 30 | 98± 9 | 151± 15 |
| 40 | 100± 3 | 148 ± 8 |
| 50 | 105± 5 | 186 ± 25 |
| 60 | 102± 8 | 166 ± 20 |
| 70 | 103± 7 | 181 ± 15 |
| 80 | 100± 10 | 173 ± 24 |
| 90 | 99± 6 | 159 ± 21 |
| 100 | 96± 5 | 174 ± 12 |
| 110 | 102± 7 | 168 ± 17 |
| 120 | 100± 5 | 173 ± 20 |
| 130 | 106± 3 | 189 ± 22 |
| 140 | 100± 2 | 177 ± 15 |
| 150 | 109± 6 | 185 ± 17 |
| 160 | 103± 8 | 171 ± 20 |
| 170 | 104± 4 | 165 ± 13 |
| 180 | 96± 7 | 159 ± 16 |
| 190 | 95± 9 | 165± 19 |
| 200 | 94± 11 | 183 ± 19 |
| 210 | 99± 9 | 192 ± 25 |
| 220 | 96± 8 | 206 ± 22 |
| 230 | 97± 11 | 175 ± 19 |
| 240 | 94± 13 | 180 ± 24 |
| 480 | 90± 14 | 182 ± 22 |
| 720 | 88± 12 | 170 ± 20 |
| 960 | 82± 16 | 167 ± 25 |
| Control group : n=5 Nefiracetam group : n=7 | | |

Effect of nefiracetam on excitatory postsynaptic potential (PS) in the brain (*in vivo*) was investigated.

A group of mice were provided. The head of each mouse was fixed, and electrodes were inserted into the granular cell layer of the hippocampal region. PS was recorded. Nefiracetam was intramuscularly injected (7.5 mg/kg). Administration of nefiracetam provided significant potentiation of the excitatory postsynaptic potential for a prolonged period. This effect was confirmed even after 960 minutes. Thus, the effect obtained *in vitro* was also confirmed in an *in vivo* experiment in which the above hippocampal slices were used. As described above, the results clearly demonstrate that the nootropic nefiracetam can influence two signal transduction pathways linked to PKA and PKC activation, providing a clue to understanding the cellular mechanism for the nootropic agents.

### INDUSTRIAL APPLICABILITY

The drug of the present invention is endowed with an action of facilitating hippocampla neurotransmission for a prolonged period (LTP-like action) without need of tetanic stimulation. Due to this action, the drug of the present invention is effective as a therapeutic drug for treatment of dementia caused by normal pressure hydrocephalus and as therapeutic and preventive drug for Alzheimer's disease. The substance of the present invention is useful as a nootropic, as it facilitates hippocampal neurotransmission for a long term without electrical stimulation, based on interaction between a long-term activation of a PKC pathway and that of presynaptic nicotinic acetylcholine receptors and on an accompanying increase in glutamate release.

The substance discovered in the present invention, i.e., substance exhibiting an LTP-like action in the absence of electrical stimulation, is an excellent candidate for a nootropic.

The substance―which facilitates hippocampal neurotransmission for a long period of time without electrical stimulation, based on interaction between a long-term activation of a PKC pathway and that of presynaptic nicotinic acetylcholine receptors and on an accompanying increase in glutamate release―is a candidate for a nootropic for Alzheimer's disease and the like, a therapeutic drug for dementia caused by normal pressure hydrocephalus.

## Claims

1. A nootropic drug which contains, as an active ingredient, a substance activating a protein kinase C-activation pathway over a long period of time.

2. A nootropic drug which contains, as an active ingredient, a substance providing an interaction of long-term activation of protein kinase C and long-term activation of a nicotinic acetylcholine receptor.

3. A nootropic drug which contains, as an active ingredient, a substance activating a protein kinase C-activation pathway over a long period of time, so as to activate a presynaptic nicotinic acetylcholine receptor based on an interaction with the protein kinase C, to thereby increase release of glutamate for a long period for the improvement of hippocampal neurotransmission over a long period of time.

4. A nootropic drug which contains, as an active ingredient, a substance evoking LTP-like action without requiring electrical stimulation.

5. A nootropic drug according to any one of Claims 1 to 3, which is a therapeutic drug for the therapy of dementia caused by chronic subdural hemorrhage.

6. A nootropic drug according to any one of Claims 1 to 3, which is a therapeutic drug for the therapy of dementia caused by hydrocephalus.

7. A nootropic drug according to any one of Claims 1 to 6, wherein the substance is 2-oxo-1-pyrrolidinylalkylcarboxylic acid amide having the following formula (1): 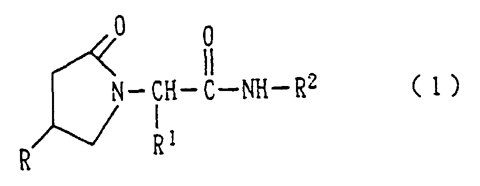 [wherein R represents a hydrogen atom or a hydroxyl group; R¹ represents a hydrogen atom or a methyl group; and R² represents a pyridyl group or a substituted phenyl group having 1 to 3 substituents which may be identical to or different from one another, wherein the substituents on the phenyl group may be
a halogen atom,
a trifluoromethyl group,
a nitro group,
an acetyl group,
a C₁₋₄ linear or branched alkyl group,
a C₁₋₄ linear or branched alkoxyl group,
a C₁₋₇ linear or branched alkylmercapto group,
a substituted alkylmercapto group represented by -S-(CH₂)ₙ-CH(R³)(R⁴) [wherein n represents 1 or 2; R³ represents a hydrogen atom or a methyl group; R⁴ represents a hydroxyl group or an amino group represented by -N(R⁸)(R⁹) (wherein R⁸ represents a hydrogen atom or a methyl group and R⁹ represents a methyl group, a benzyl group, or a substituted benzyl group, or R⁸ and R⁹ may be linked to each other and form a substituted pyrrolidine ring together with N in the formula)],
a sulfonyl group represented by -SO₂R⁵ (wherein R⁵ represents an amino group or a C₁₋₃ alkyl group), or
a substituted aminoethoxycarbonyl group represented by -COO(CH₂)₂-N(R⁶)(R⁷) (wherein each of R⁶ and R⁷ represents a hydrogen atom, a methyl group, or an ethyl group)],
or a pharmaceutically acceptable acid addition salt thereof.

8. A nootropic drug according to any one of Claims 1 to 6, wherein the substance is selected from the group consisting of the following compounds:
(1) 2-oxo-1-pyrrolidinylacetic acid 2,6-dimethylanilide;
(2) 4-hydroxy-2-oxo-1-pyrrolidinylacetic acid 2,6-diethylanilide;
(3) 4-hydroxy-2-oxo-1-pyrrolidinylacetic acid 2,6-dimethylanilide;
(4) 2-[2-oxopyrrolidinyl]propionic acid N-3-pyridinylamide;
(5) 2-oxo-1-pyrrolidinylacetic acid 4-isopropylmercaptoanilide;
(6) 2-[2-oxo-1-pyrrolidinyl]-1-propionic acid-4-(2-butylmercapto)anilide;
(7) 2-[2-oxo-1-pyrrolidinyl]propionic acid-4-isopropylanilide;
(8) 2-[2-oxo-1-pyrrolidinyl]propionic acid-2,4-dimethylanilide;
(9) 2-[2-oxo-1-pyrrolidinyl]propionic acid-2,4,6-trimethylanilide;
(10) 2-[2-oxo-1-pyrrolidinyl]propionic acid-2-methoxy-5-methylanilide;
(11) 2-[2-oxo-1-pyrrolidinyl]propionic acid-2,6-dichloroanilide;
(12) 2-pyrrolidone-acetamide;
(13) 1-anisoyl-2-pyrrolidinone; and
(14) 4-hydroxy-2-oxo-1-pyrrolidineacetamide.

9. A nootropic drug according to any one of Claims 1 to 6, wherein the substance is selected from the group consisting of 2-oxo-1-pyrrolidinylacetic acid 2,6-dimethylanilide, 2-pyrrolidone-acetamide, 1-anisoyl-2-pyrrolidinone, and 4-hydroxy-2-oxo-1-pyrrolidineacetamide.

10. A nootropic drug according to any one of Claims 1 to 6, wherein the substance is nefiracetam.

11. A long-term cerebral neurotransmission facilitating agent containing, as an active ingredient, a compound represented by the following formula (1) : 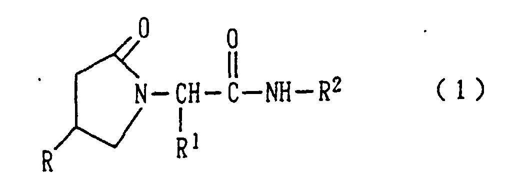 [wherein R represents a hydrogen atom or a hydroxyl group; R¹ represents a hydrogen atom or a methyl group; and R² represents a pyridyl group or a substituted phenyl group having 1 to 3 substituents which may be identical to or different from one another, wherein the substituents on the phenyl group may be
a halogen atom,
a trifluoromethyl group,
a nitro group,
an acetyl group,
a C₁₋₄ linear or branched alkyl group,
a C₁₋₄ linear or branched alkoxyl group,
a C₁₋₇ linear or branched alkylmercapto group,
a substituted alkylmercapto group represented by -S-(CH₂)ₙ-CH(R³)(R⁴) [wherein n represents 1 or 2; R³ represents a hydrogen atom or a methyl group; R⁴ represents a hydroxyl group, or an amino group represented by -N(R⁸)(R⁹) (wherein R⁸ represents a hydrogen atom or a methyl group and R⁹ represents a methyl group, a benzyl group, or a substituted benzyl group, or R⁸ and R⁹ may be linked to each other and form a substituted pyrrolidine ring together with N in the formula)],
a sulfonyl group represented by -SO₂R⁵ (wherein R⁵ represents an amino group or a C₁₋₃ alkyl group), or
a substituted aminoethoxycarbonyl group represented by -COO(CH₂)₂-N(R⁶)(R⁷) (wherein each of R⁶ and R⁷ represents a hydrogen atom, a methyl group, or an ethyl group)],
or a pharmaceutically acceptable acid-addition salt thereof.

12. A cerebral neurotransmission facilitating agent containing a compound selected from the group consisting of the following compounds (1) through (14):
(1) 2-oxo-1-pyrrolidinylacetic acid 2,6-dimethylanilide;
(2) 4-hydroxy-2-oxo-1-pyrrolidinylacetic acid 2,6-diethylanilide;
(3) 4-hydroxy-2-oxo-1-pyrrolidinylacetic acid 2,6-dimethylanilide;
(4) 2-[2-oxopyrrolidinyl]propionic acid N-3-pyridinylamide;
(5) 2-oxo-1-pyyrolidinylacetic acid 4-isopropylmercaptoanilide;
(6) 2-[2-oxo-1-pyrrolidinyl]-1-propionic acid 4-(2-butylmercapto)anilide;
(7) 2-[2-oxo-1-pyrrolidinyl]propionic acid 4-isopropylanilide;
(8) 2-[2-oxo-1-pyrrolidinyl]propionic acid 2,4-dimethylanilide;
(9) 2-[2-oxo-1-pyrrolidinyl]propionic acid 2,4,6-methoxy-5-methylanilide;
(10) 2-[2-oxo-1-pyrrolidinyl]propionic acid 2-methoxy-5-methylanilide;
(11) 2-[2-oxo-1-pyrrolidinyl]propionic acid 2,6-dichloroanilide;
(12) 2-pyrrolidone-acetamide;
(13) 1-anisoyl-2-pyrrolidinone; and
(14) 4-hydroxy-2-oxo-1-pyrrolidine-acetamide.

13. A long-term activating agent for a protein kinase C activation pathway, which contains nefiracetam as an active ingredient.

14. An LTP-like action inducer requiring no electrical stimulation, which contains nefiracetam as an active ingredient.

15. A long-term enhancer for glutamate release based on interaction between a long-term activation of a protein kinase C activation pathway and that of presynaptic nicotinic acetylcholorine receptors, which contains nefiracetam as an active ingredient.

16. A long-term brain neurotransmission enhancer based on a long-term activation of a protein kinase C activation pathway and a long-term activation of presynaptic nicotinic acetylcholine receptors, which enhancer contains nefiracetam as an active ingredient.

17. A therapeutic drug for the therapy of dementia caused by chronic cerebral subdural hemorrhage, the therapeutic drug being based on evoking an LTP-like action requiring no electrical stimulation and provided by activation of a protein kinase C activation pathway, and containing nefiracetam as an active ingredient.

18. A therapeutic drug containing nefiracetam as an active ingredient and used for dementia caused by hydrocephalus, the therapeutic drug being based on evoking an LTP-like action requiring no electrical stimulation and provided by activation of a protein kinase C activation pathway.

19. A nootropic drug containing nefiracetam as an active ingredient, which facilitates hippocampal neurotransmission for a prolonged period of time based on an increase in the amount of released glutamate for a long period of time, which increase being provided by interaction of activation of a protein kinase C activation pathway and long-term activation of presynaptic nicotinic acetylcholine receptors.

20. A long-term hippocampal neurotransmission improving agent containing nefiracetam as an active ingredient, which increases the amount of released glutamate for a prolonged period of time, through activation of a protein kinase C activation pathway and activation of presynaptic nicotinic acetylcholine receptors.

21. A long-term enhancer for release of glutamate, which contains nefiracetam as an active ingredient.

22. Use, in manufacture of a nootropic drug, of a substance which activates a protein kinase C-activation pathway over a long period of time.

23. Use, in manufacture of a nootropic drug, of a substance which provides an interaction of long-term activation of protein kinase C and long-term activation of a nicotinic acetylcholine receptor.

24. Use, in manufacture of a nootropic drug, of a substance which activates a protein kinase C-activation pathway over a long period of time, so as to activate a presynaptic nicotinic acetylcholine receptor based on an interaction with the protein kinase C, to thereby increase release of glutamate for a long period for the improvement of hippocampal neurotransmission over a long period of time.

25. Use, in manufacture of a nootropic drug, of a substance which evokes an LTP-like action without requiring electrical stimulation.

26. Use according to any one of Claims 22 to 25, wherein the nootropic drug is a therapeutic drug for the therapy of dementia caused by chronic subdural hemorrhage.

27. Use according to any one of Claims 22 to 25, wherein the nootropic drug is a therapeutic drug for the therapy of dementia caused by hydrocephalus.

28. Use according to any one of Claims 22 to 27, wherein the substance is 2-oxo-1-pyrrolidinylalkylcarboxylic amide represented by the following formula (1) : 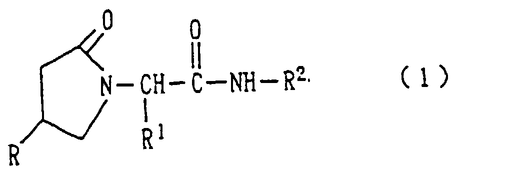 [wherein R represents a hydrogen atom or a hydroxyl group; R¹ represents a hydrogen atom or a methyl group; and R² represents a pyridyl group or a substituted phenyl group having 1 to 3 substituents which may be identical to or different from one another, wherein the substituents on the phenyl group may be
a halogen atom,
a trifluoromethyl group,
a nitro group,
an acetyl group,
a C₁₋₄ linear or branched alkyl group,
a C₁₋₄ linear or branched alkoxyl group,
a C₁₋₇ linear or branched alkylmercapto group,
a substituted alkylmercapto group represented by -S-(CH₂)ₙ-CH(R³)(R⁴) [wherein n represents 1 or 2; R³ represents a hydrogen atom or a methyl group; R⁴ represents a hydroxyl group or an amino group represented by -N(R⁸)(R⁹) (wherein R⁸ represents a hydrogen atom or a methyl group and R⁹ represents a methyl group, a benzyl group, or a substituted benzyl group, or R⁸ and R⁹ may be linked to each other and form a substituted pyrrolidine ring together with N in the formula)],
a sulfonyl group represented by -SO₂R⁵ (wherein R⁵ represents an amino group or a C₁₋₃ alkyl group), or
a substituted aminoethoxycarbonyl group represented by -COO(CH₂)₂-N(R⁶)(R⁷) (wherein each of R⁶ and R⁷ represents a hydrogen atom, a methyl group, or an ethyl group)],
or a pharmaceutically acceptable acid-addition salt thereof.

29. Use according to any one of Claims 22 to 27, wherein the substance is selected from the group consisting of the following compounds:
(1) 2-oxo-1-pyrrolidinylacetic acid 2,6-dimethylanilide;
(2) 4-hydroxy-2-oxo-1-pyrrolidinylacetic acid 2,6-diethylanilide;
(3) 4-hydroxy-2-oxo-1-pyrrolidinylacetic acid 2,6-dimethylanilide;
(4) 2-[2-oxopyrrolidinyl]propionic acid N-3-pyridinylamide;
(5) 2-oxo-1-pyyrolidinylacetic acid 4-isopropylmercaptoanilide;
(6) 2-[2-oxo-1-pyrrolidinyl]-1-propionic acid 4-(2-butylmercapto)anilide;
(7) 2-[2-oxo-1-pyrrolidinyl]propionic acid 4-isopropylanilide;
(8) 2-[2-oxo-1-pyrrolidinyl]propionic acid 2,4-dimethylanilide;
(9) 2-[2-oxo-1-pyrrolidinyl]propionic acid 2,4,6-methoxy-5-methylanilide;
(10) 2-[2-oxo-1-pyrrolidinyl]propionic acid 2-methoxy-5-methylanilide;
(11) 2-[2-oxo-1-pyrrolidinyl]propionic acid 2,6-dichloroanilide;
(12) 2-pyrrolidone-acetamide;
(13) 1-anisoyl-2-pyrrolidinone; and
(14) 4-hydroxy-2-oxo-1-pyrrolidine-acetamide.

30. Use according to any one of Claims 22 to 27, wherein the substance is selected from the group consisting of 2-oxo-1-pyrrolidinylacetic acid 2,6-dimethylanilide, 2-pyrrolidone-acetamide, 1-anisoyl-2-pyrrolidinone, and 4-hydroxy-2-oxo-1-pyrrolidineacetamide.

31. Use according to any one of Claims 22 to 27, wherein the substance is nefiracetam.

32. Use, in manufacture of a long-term cerebral neurotransmission facilitating agent, of 2-oxo-1-pyrrolidinylalkylcarboxylic acid amide having the following formula (1): 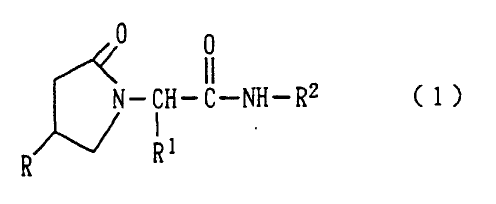 [wherein R represents a hydrogen atom or a hydroxyl group; R¹ represents a hydrogen atom or a methyl group; and R² represents a pyridyl group or a substituted phenyl group having 1 to 3 substituents which may be identical to or different from one another, wherein the substituents on the phenyl group may be
a halogen atom,
a trifluoromethyl group,
a nitro group,
an acetyl group,
a C₁₋₄ linear or branched alkyl group,
a C₁₋₄ linear or branched alkoxyl group,
a C₁₋₇ linear or branched alkylmercapto group,
a substituted alkylmercapto group represented by -S-(CH₂)ₙ-CH(R³)(R⁴) [wherein n represents 1 or 2; R³ represents a hydrogen atom or a methyl group; R⁴ represents a hydroxyl group or an amino group represented by -N(R⁸)(R⁹) (wherein R⁸ represents a hydrogen atom or a methyl group and R⁹ represents a methyl group, a benzyl group, or a substituted benzyl group, or R⁸ and R⁹ may be linked to each other and form a substituted pyrrolidine ring together with N in the formula)],
a sulfonyl group represented by -SO₂R⁵ (wherein R⁵ represents an amino group or a C₁₋₃ alkyl group), or
a substituted aminoethoxycarbonyl group represented by -COO(CH₂)₂-N(R⁶)(R⁷) (wherein each of R⁶ and R⁷ represents a hydrogen atom, a methyl group, or an ethyl group)],
or a pharmaceutically acceptable acid addition salt thereof.

33. Use, in manufacture of a long-term cerebral neurotransmission facilitating agent, of a compound selected from the group consisting of the following compounds 1. through 14:
(1) 2-oxo-1-pyrrolidinylacetic acid 2,6-dimethylanilide;
(2) 4-hydroxy-2-oxo-1-pyrrolidinylacetic acid 2,6-diethylanilide;
(3) 4-hydroxy-2-oxo-1-pyrrolidinylacetic acid 2,6-dimethylanilide;
(4) 2-[2-oxopyrrolidinyl]propionic acid N-3-pyridinylamide;
(5) 2-oxo-1-pyyrolidinylacetic acid 4-isopropylmercaptoanilide;
(6) 2-[2-oxo-1-pyrrolidinyl]-1-propionic acid 4-(2-butylmercapto)anilide;
(7) 2-[2-oxo-1-pyrrolidinyl]propionic acid 4-isopropylanilide;
(8) 2-[2-oxo-1-pyrrolidinyl]propionic acid 2,4-dimethylanilide;
(9) 2-[2-oxo-1-pyrrolidinyl]propionic acid 2,4,6-methoxy-5-methylanilide;
(10) 2-[2-oxo-1-pyrrolidinyl]propionic acid 2-methoxy-5-methylanilide;
(11) 2-[2-oxo-1-pyrrolidinyl]propionic acid 2,6-dichloroanilide;
(12) 2-pyrrolidone-acetamide;
(13) 1-anisoyl-2-pyrrolidinone; and
(14) 4-hydroxy-2-oxo-1-pyrrolidine-acetamide.

34. Use of nefiracetam in manufacture of a long-term activating agent for a protein kinase C activation pathway.

35. Use of nefiracetam in manufacture of an inducer of an LTO-like action without requiring electrical stimulation.

36. Use of nefiracetam in manufacture of a long-term enhancer for glutamate release based on interaction between a long-term activation of a protein kinase C activation pathway and that of presynaptic nicotinic acetylcholorine receptors.

37. Use of nefiracetam in manufacture of a long-term cerebral neurotransmission facilitator based on a long-term activation of a protein kinase C activation pathway and a long-term activation of presynaptic nicotinic acetylcholine receptors.

38. Use of nefiracetam in manufacture of a therapeutic drug for the therapy of dementia caused by chronic cerebral subdural hemorrhage, the therapeutic drug being based on evoking an LTP-like action requiring no electrical stimulation and provided by activation of a protein kinase C activation pathway.

39. Use of nefiracetam in manufacture of a therapeutic drug for the therapy of dementia caused by hydrocephalus, the therapeutic drug being based on evoking an LTP-like action requiring no electrical stimulation and provided by activation of a protein kinase C activation pathway.

40. Use of nefiracetam in manufacture of a nootropic whose effect is exerted based on long-term facilitation of hippocampal neurotransmission obtained through an increase in the amount of released glutamate for a long period of time, which increase being provided by interaction of activation of a protein kinase C activation pathway and long-term activation of presynaptic nicotinic acetylcholine receptors.

41. Use of nefiracetam in manufacture of a long-term hippocampal neurotransmission facilitator whose effect is exerted based on an increase in the amount of released glutamate for a prolonged period of time, through activation of a protein kinase C activation pathway and activation of presynaptic nicotinic acetylcholine receptors.

42. Use of nefiracetam in manufacture of a long-term enhancer for release of glutamate.

43. A treatment method for enhancing cognition, characterized by administering to a subject a substance which activates a protein kinase C-activation pathway over a long period of time.

44. A treatment method for enhancing cognition, characterized by administering to a subject a substance which provides an interaction of long-term activation of protein kinase C and long-term activation of a nicotinic acetylcholine receptor.

45. A treatment method for enhancing cognition, characterized by administering to a subject a substance which activates a protein kinase C-activation pathway over a long period of time, so as to activate a presynaptic nicotinic acetylcholine receptor based on an interaction with the protein kinase C, to thereby increase release of glutamate for a long period for the improvement of hippocampal neurotransmission over a long period of time.

46. A treatment method for enhancing cognition, characterized by administering to a subject a substance which evokes an LTP-like action without requiring electrical stimulation.

47. A method according to any one of claims 43 to 46, wherein the treatment for engancing cognition is directed to therapy of dementia caused by chronic subdural hemorrhage.

48. A method according to any one of claims 43 to 46, wherein the treatment for engancing cognition is directed to therapy of dementia caused by hydrocephalus.

49. A method according to any one of claims 43 to 48, wherein the substance is 2-oxo-1-pyrrolidinylalkyl-carboxylic acid amide having the following formula (1): 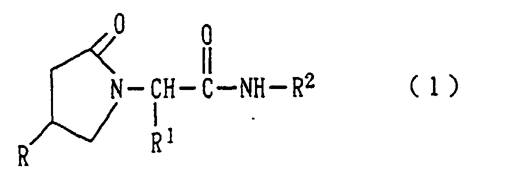 [wherein R represents a hydrogen atom or a hydroxyl group; R¹ represents a hydrogen atom or a methyl group; and R² represents a pyridyl group or a substituted phenyl group having 1 to 3 substituents which may be identical to or different from one another, wherein the substituents on the phenyl group may be
a halogen atom,
a trifluoromethyl group,
a nitro group,
an acetyl group,
a C₁₋₄ linear or branched alkyl group,
a C₁₋₄ linear or branched alkoxyl group,
a C₁₋₇ linear or branched alkylmercapto group,
a substituted alkylmercapto group represented by -S-(CH₂)ₙ-CH(R³)(R⁴) [wherein n represents 1 or 2; R³ represents a hydrogen atom or a methyl group; R⁴ represents a hydroxyl group or an amino group represented by -N(R⁸)(R⁹) (wherein R⁸ represents a hydrogen atom or a methyl group and R⁹ represents a methyl group, a benzyl group, or a substituted benzyl group, or R⁸ and R⁹ may be linked to each other and form a substituted pyrrolidine ring together with N in the formula)],
a sulfonyl group represented by -SO₂R⁵ (wherein R⁵ represents an amino group or a C₁₋₃ alkyl group), or
a substituted aminoethoxycarbonyl group represented by -COO(CH₂)₂-N(R⁶)(R⁷) (wherein each of R⁶ and R⁷ represents a hydrogen atom, a methyl group, or an ethyl group)],
or a pharmaceutically acceptable acid addition salt thereof.

50. A method according to any one of claims 43 to 48, wherein the substance is selected from the group consisting of the following compounds:
(1) 2-oxo-1-pyrrolidinylacetic acid 2,6-dimethylanilide;
(2) 4-hydroxy-2-oxo-1-pyrrolidinylacetic acid 2,6-diethylanilide;
(3) 4-hydroxy-2-oxo-1-pyrrolidinylacetic acid 2,6-dimethylanilide;
(4) 2-[2-oxopyrrolidinyl]propionic acid N-3-pyridinylamide;
(5) 2-oxo-1-pyrrolidinylacetic acid 4-isopropylmercaptoanilide;
(6) 2-[2-oxo-1-pyrrolidinyl]-1-propionic acid-4-(2-butylmercapto)anilide;
(7) 2-[2-oxo-1-pyrrolidinyl]propionic acid-4-isopropylanilide;
(8) 2-[2-oxo-1-pyrrolidinyl]propionic acid-2,4-dimethylanilide;
(9) 2-[2-oxo-1-pyrrolidinyl]propionic acid-2,4,6-trimethylanilide;
(10) 2-[2-oxo-1-pyrrolidinyl]propionic acid-2-methoxy-5-methylanilide;
(11) 2-[2-oxo-1-pyrrolidinyl]propionic acid-2,6-dichloroanilide;
(12) 2-pyrrolidone-acetamide;
(13) 1-anisoyl-2-pyrrolidinone; and
(14) 4-hydroxy-2-oxo-1-pyrrolidineacetamide.

51. A method according to any one of claims 43 to 48, wherein the substance is selected from the group consisting of 2-oxo-1-pyrrolidinylacetic acid 2,6-dimethylanilide, 2-pyrrolidone-acetamide, 1-anisoyl-2-pyrrolidinone, and 4-hydroxy-2-oxo-1-pyrrolidineacetamide.

52. A method according to any one of claims 43 to 48, wherein the substance is nefiracetam.

53. A treatment method for providing long-term cerebral neurotransmission facilitation, characterized by administering to a subject 2-oxo-1-pyrrolidinylalkylcarboxylic acid amide represented by the following formula (1): 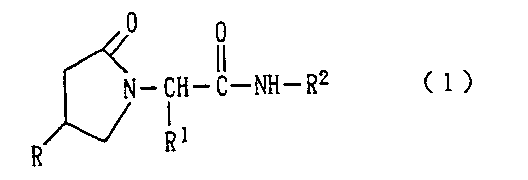 [wherein R represents a hydrogen atom or a hydroxyl group; R¹ represents a hydrogen atom or a methyl group; and R² represents a pyridyl group or a substituted phenyl group having 1 to 3 substituents which may be identical to or different from one another, wherein the substituents on the phenyl group may be
a halogen atom,
a trifluoromethyl group,
a nitro group,
an acetyl group,
a C₁₋₄ linear or branched alkyl group,
a C₁₋₄ linear or branched alkoxyl group,
a C₁₋₇ linear or branched alkylmercapto group,
a substituted alkylmercapto group represented by -S-(CH₂)ₙ-CH(R³)(R⁴) [wherein n represents 1 or 2; R³ represents a hydrogen atom or a methyl group; R⁴ represents a hydroxyl group or an amino group represented by -N(R⁸)(R⁹) (wherein R⁸ represents a hydrogen atom or a methyl group and R⁹ represents a methyl group, a benzyl group, or a substituted benzyl group, or R⁸ and R⁹ may be linked to each other and form a substituted pyrrolidine ring together with N in the formula)],
a sulfonyl group represented by -SO₂R⁵ (wherein R⁵ represents an amino group or a C₁₋₃ alkyl group), or
a substituted aminoethoxycarbonyl group represented by -COO(CH₂)₂-N(R⁶)(R⁷) (wherein each of R⁶ and R⁷ represents a hydrogen atom, a methyl group, or an ethyl group)],
or a pharmaceutically acceptable acid-addition salt thereof.

54. A treatment method for providing long-term cerebral neurotransmission facilitation, characterized by administering to a subject a compound selected from the group consisting of the following compounds (1) through (14):
(1) 2-oxo-1-pyrrolidinylacetic acid 2,6-dimethylanilide;
(2) 4-hydroxy-2-oxo-1-pyrrolidinylacetic acid 2,6-diethylanilide;
(3) 4-hydroxy-2-oxo-1-pyrrolidinylacetic acid 2,6-dimethylanilide;
(4) 2-[2-oxopyrrolidinyl]propionic acid N-3-pyridinylamide;
(5) 2-oxo-1-pyyrolidinylacetic acid 4-isopropylmercaptoanilide;
(6) 2-[2-oxo-1-pyrrolidinyl]-1-propionic acid 4-(2-butylmercapto)anilide;
(7) 2-[2-oxo-1-pyrrolidinyl]propionic acid 4-isopropylanilide;
(8) 2-[2-oxo-1-pyrrolidinyl]propionic acid 2,4-dimethylanilide;
(9) 2-[2-oxo-1-pyrrolidinyl]propionic acid 2,4,6-methoxy-5-methylanilide;
(10) 2-[2-oxo-1-pyrrolidinyl]propionic acid 2-methoxy-5-methylanilide;
(11) 2-[2-oxo-1-pyrrolidinyl]propionic acid 2,6-dichloroanilide;
(12) 2-pyrrolidone-acetamide;
(13) 1-anisoyl-2-pyrrolidinone; and
(14) 4-hydroxy-2-oxo-1-pyrrolidine-acetamide.

55. A treatment method for providing long-term activation of a protein kinase C activation pathway, characterized by administering nefiracetam to a subject.

56. A treatment method for evoking an LTP-like action requiring no electrical stimulation, characterized by administering nefiracetam to a subject.

57. A treatment method for providing a long-term increase in glutamate release based on interaction between a long-term activation of a protein kinase C activation pathway and that of presynaptic nicotinic acetylcholorine receptors, characterized by administering nefiracetam to a subject.

58. A treatment method for providing a long-term facilitation of cerebral neuritransmission based on a long-term activation of a protein kinase C activation pathway and a long-term activation of presynaptic nicotinic acetylcholine receptors.

59. A treatment method for the therapy of dementia caused by chronic cerebral subdural hemorrhage, characterized in that the method includes evoking of an LTP-like action in the absence of electrical stimulation through activation of a protein kinase C activation pathway, and also includes administration of nefiracetam to a subject.

60. A treatment method for the therapy of dementia caused by hydrocephalus, characterized in that the method includes evoking of an LTP-like action in the absence of electrical stimulation caused by activation of a protein kinase C activation pathway.

61. A treatment method for enhancing cognition, characterized in that the method includes long-term facilitation of hippocampal neurotransmission caused by an increase in the amount of released glutamate for a long period of time, which increase being provided by interaction of activation of a protein kinase C activation pathway and long-term activation of presynaptic nicotinic acetylcholine receptors, and includes administration of nefiracetam to a subject.

62. A treatment method for long-term facilitation of hippocampal neurotransmission, characterized in that the method includes activation of a protein kinase C activation pathway and activation of presynaptic nicotinic acetylcholine receptors so as to enhance the amount of released glutamate for a prolonged period of time, and administration of nefiracetam to a subject.

63. A treatment method for increasing the amount of glutamate for a prolonged period, characterized in that the method includes administration of nefiracetam to a subject.
